# EUROPÄISCHE PATENTANMELDUNG

(11) **EP 4 736 754 A2**
(43) Veröffentlichungstag der Anmeldung: **06.05.2026**
(21) Anmeldenummer: 26159473.3
(22) Anmeldetag: 10.06.2021
(51) Int. Cl.: A61B 5/0245

(54) **ÜBERWACHUNGSSYSTEM**

(30) Priorität: 29.06.2020 DE 102020117040; 04.05.2021 DE 102021111431
(62) Teilanmeldung aus: 21732864.0
(71) Anmelder: Dräger Safety AG & Co. KGaA, 23560 Lübeck (DE)
(72) Erfinder: Osterloh, Christoph, 23558 Lübeck (DE); Gerder, Henning, 23558 Lübeck (DE)

(57) **Zusammenfassung**

Es wird ein Überwachungssystem (100') für Piloten oder Copiloten (99) von Luftfahrzeugen beschrieben. Mittels einer Sensorik (60) erfolgt eine messtechnische Erfassung von Gaskonzentrationen.

Zudem ist in oder an dem Überwachungssystem (100) ein Beschleunigungssensor (61) vorgesehen, welcher dem Piloten (99) oder dem Copiloten Eingaben als Eingabeelement, beispielsweise in einer Funktionalität als Paniktaste oder zu einer Markierung von Situationen am Überwachungssystem (100) zur Verfügung steht.

## Beschreibung

Die vorliegende Erfindung betrifft ein Überwachungssystem mit einer Eingabefunktionalität. Das Überwachungssystem mit Eingabefunktionalität ist zu einer Überwachung einer Atemgasversorgung für fliegerisches Personal von Luftfahrzeugen oder Fluggeräten vorgesehen und geeignet ausgebildet. Die Ausprägung der Eingabefunktionalität ermöglicht dem fliegerischen Personal eine Handhabung und Bedienung des Überwachungssystems während eines Flugeinsatzes im Luftfahrzeug.

Unter Luftfahrzeugen oder Fluggeräten sind Flugzeuge oder Hubschrauber der zivilen oder militärischen Luftfahrt, sowie auch ultraschnelle Flugzeuge nahe dem Bereich oder oberhalb des Bereiches der Überschallgeschwindigkeit, zu verstehen.

Insbesondere Flüge mit Strahlflugzeugen (Jets) mit Überschallgeschwindigkeiten und/oder in Flughöhen oberhalb von 15.000 Metern oberhalb des Meeresbodens stellen große Anforderungen an das fliegerische Personal, insbesondere an die Piloten von Strahlflugzeugen, hinsichtlich der Flugtauglichkeit mit körperlicher und geistiger Fitness, Aufmerksamkeit, Konzentrationsfähigkeit und Wachsamkeit dar. Damit die körperliche und geistige Fitness und die zum Führen des Fluggerätes erforderliche Wachsamkeit jederzeit in großen Höhen, rasanten Flugmanövern oder Fluglagen, wie beispielsweise Kurvenflug, Sturzflug, Rückenflug mit hohen Geschwindigkeiten (> Mach 1) und mit Beschleunigungen oberhalb oder gar mehrfacher Erdbeschleunigung sowie auch Betankungen in der Luft, gewährleistet ist, ist neben einer verlässlichen Ausstattung des Flugzeugs auch eine gesicherte Versorgung des Flugzeugführers mit einwandfreier und gesundheitlich unbedenklicher Atemluft sehr wesentlich.

Zur Versorgung von Luftfahrzeugführern, Flugzeugführern, Piloten, Copiloten mit Atemluft bzw. Atemgas werden beispielsweise Systeme eingesetzt, welche - zumeist aufbereitete oder klimatisierte und gefilterte - Außenluft aus der Umgebung als Atemgasquelle verwenden, es werden aber auch Systeme eingesetzt, in welchen zusätzlicher Sauerstoff der Atemluft bzw. dem Atemgas zugefügt wird.

Der Sauerstoff kann dabei, beispielsweise unter hohem Druck (< 200 bar), mittels Drucksauerstoffflaschen im Flugzeug mitgeführt werden und mittels geeigneter Apparaturen zur Druckminderung auf atembaren Druck herabgesetzt werden oder zum direkten Verbrauch im Einsatz durch einen chemischen Sauerstoffgenerator, beispielsweise aus mitgeführtem Natriumchlorat, in einem chemischen Prozess erzeugt werden. Vielfach ist es dem Flugzeugführer, Piloten oder Copiloten dabei ermöglicht, die Dosierung oder Versorgung mit Sauerstoff eigenständig zu aktivieren und/oder Menge und/oder eine Konzentration von Sauerstoff und/oder eine Zusammensetzung des Atemgases eigenständig einzustellen oder vorzugeben. Die Atemluft-/Atemgasversorgung kann dabei direkt aus der Luft von Kabine oder Cockpit erfolgen, es kann aber auch ein Schlauchsystem mit Mund-/Nasen-Maske zu direkter Zufuhr und/oder Abfuhr von Atemluft/Atemgas zum Flugzeugführer, Piloten oder Copiloten zum Einsatz kommen. In jedem Fall ist es erforderlich, dass die Bordtechnik des Luftfahrzeuges oder Fluggerätes die Flugzeugführer, Piloten oder Copiloten im Einsatz mit einwandfreiem und gesundheitlich unbedenklichem Atemgas versorgt. Dazu gehört einerseits, dass die qualitative und quantitative Zusammensetzung des Atemgases, insbesondere die Anteile an Sauerstoff und/oder Kohlenstoffdioxid, in dem Atemgas in einem gesundheitlich unbedenklichen Bereich liegen. In der natürlichen Atmosphäre macht - neben Anteilen von Stickstoff und Edelgasen - der Sauerstoff (O₂) einen Anteil von 21% Vol. aus. Der Anteil an Kohlenstoffdioxid (CO₂) liegt aktuell in der natürlichen Atmosphäre im weltweiten Mittel unterhalb von 0,05 % Vol. Nach Empfehlungen der amerikanischen Luftfahrtbundesbehörde (FAA) stellt eine Kohlenstoffdioxidkonzentration in Luftfahrzeugen von 30.000 ppm - entsprechend 3% Vol. CO₂- den höchsten zulässigen Wert dar.

Die amerikanische Gesellschaft für Heizung, Kühlung und Klimatisierung (ASHRAE) empfiehlt als Obergrenze eine Kohlenstoffdioxidkonzentration von 1.000 ppm - entsprechend 0,1 % Vol. CO₂. Somit ist auch für die Bereitstellung von Atemgas für Flugzeugführer, Piloten oder Copiloten für den Anteil an Sauerstoff eine Konzentration oberhalb von 21% Vol. und für den Anteil von Kohlenstoffdioxid zumindest gemäß der Empfehlungen der amerikanischen Luftfahrtbundesbehörde (FAA) bzw. gemäß der Empfehlungen der amerikanischen Gesellschaft für Heizung, Kühlung und Klimatisierung (ASHRAE) eine Obergrenze von 0,1 % Vol. CO₂ im Einsatz anzustreben.

Als wissenschaftlich gesichert gilt, dass Konzentrationen an Kohlenstoffdioxid oberhalb von 1% - 3% Vol. eine Kohlenstoffdioxidvergiftung hervorrufen können, welche beispielsweise durch Übelkeit, Kopfschmerzen und Schwindel gekennzeichnet ist. Konzentrationen an Kohlenstoffdioxid oberhalb von 12% sind unmittelbar tödlich.

Auch eine Unterversorgung mit Sauerstoff kann insbesondere für Flugzeugführer, Piloten oder Copiloten gesundheitlich bedenklich sein, da bei Unterversorgung mit Sauerstoff der Sauerstoffpartialdruck im Blut herabgesetzt werden kann, es tritt ein sogenannter hypoxischer Zustand (Hypoxie) ein.

Eine solche Erniedrigung des arteriellen Sauerstoffpartialdrucks im Blut - auch als hypoxämische Hypoxie (Hypoxämie) bezeichnet - ergibt sich oftmals bei einem Aufenthalt in großen Höhen. Symptome von Hypoxie sind beispielsweise: Angst und Unruhe, Dyspnoe, Zyanose, Tachykardie, Blutdruckanstieg, Verwirrtheit, Schwindel, Bradykardie bis hin zum Herzstillstand.

Die US 10,561,863 B1 zeigt eine am menschlichen Körper tragbare Messeinrichtung für physiologische oder biologische Parameter bezüglich Metabolismus, Herz-/ Kreislaufsystem, Atemsystem mit Sauerstoffversorgung, Sauerstoffsättigung und Funktionen der Lunge. Weitere Aspekte betreffen auch ein System zu einer Zusatz- oder Notgasversorgung (OBOGS- System = on board oxygen generation system) mit Sauerstoff für ein Luftfahrzeug.

Aus der US 10,786,693 B1 ist ein tragbares Gerät zu einem Bio-Monitoring von physiologischen Messgrößen zur Bestimmung von Stoffwechsel-, Lungen- und Herzfunktion und Sauerstoffsättigung bekannt. Das Gerät überwacht an einer Atemmaske angeordnet auf nicht-invasive Weise ein physiologisches Profil einer Person und ist in der Lage, physiologische Veränderungen zu erkennen, das Auftreten von Symptomen vorherzusagen oder zu alarmieren.

Aus der DE 10 2019 004 760 A1 ist ein Patientenmodul zu einer Beatmung eines Patienten bekannt, welches an eine Druckquelle und an eine Patientenschnittstelle koppelbar ist. Das Patientenmodul weist ein Gehäuse mit einer Ventilsektion und einen HME-Filter auf. Der HME-Filter verhindert eine Bildung von Kondensat innerhalb der Ventilsektion.

Die US20090320380 A1 beschreibt ein Verfahren zur Regulierung eines statischen Innendrucks mittels einer Bestimmung eines externen dynamischen Drucks und eines Vergleichs des statischen Innendrucks mit der Summe des externen statischen Drucks. Vergleichs des statischen Innendrucks mit der Summe des externen statischen Drucks.

Aus der EP3287173A1 ist eine Vorrichtung und ein Verfahren zur Überwachung von Einatemgas bekannt. Es wird ein Druckniveau der gesamten eingeatmeten Atemluft und ein Sauerstoffpartialdruck in der eingeatmeten Atemluft während der Zuströmung der Atemluft in eine Gesichtsmaske einer Person ermittelt und daraus ein Partialdruck des Sauerstoffs in der Lunge der Person abgeschätzt.

Aus der US2007181129A ist eine Atemmaske mit Anzeigevorrichtung bekannt,
die ausgebildet ist, Daten und/oder Informationen für Flugzeugführer, Piloten oder Copiloten visuell bereitzustellen. Die Anzeigevorrichtung ist als ein sogenanntes Head-Up-Display ausgestaltet. Es erfolgt dabei eine innenseitige Projektion der Daten und/oder Informationen auf das Visier in das Blickfeld
des Flugzeugführers, Piloten oder Copiloten.

Aus der US7391574B2 ist ein weiteres Head-Up-Display bekannt.

Aus der US2016253561A ist eine Gesichtsmaske mit einer Erfassungseinrichtung von Umgebungstemperaturen und deren Anzeige bzw. Visualisierung bekannt.

Aus der US2019118008A ist eine Anzeigevorrichtung für eine Gesichtsmaske in einer Ausgestaltung als ein sogenanntes In-Mask-Display bekannt.

Aus der US8210175B ist eine Vorrichtung zur Sauerstoffversorgung beispielsweise nach dem Prinzip der Druckwechseladsorption für ein Flugzeug bekannt.

Sauerstoff wird zudem aus einem Sauerstoffvorrat bereitgestellt. Es erfolgt eine Aufbereitung der Luft mit Molekularsiebbetten, welche zu Beginn des Betriebes mit Sauerstoff aus dem Sauerstoffvorrat gespült wurden.

Aus der US7407528B, US2004245390A und US7264647B sind weitere Vorrichtungen zur Sauerstoffversorgung in Flugzeugen bekannt.

Aus der DE102010014222B4 ist eine Druckluftüberwachungseinrichtung zu einer Überwachung von Druckluft mit einer Messluftleitung zur kontinuierlichen Entnahme von Druckluft aus einer Druckluftversorgungsleitung und mit wenigstens einem Sensor zur kontinuierlichen Erfassung wenigstens eines Parameters der Druckluft bekannt.

Als Sensoren zur kontinuierlichen Erfassung wenigstens eines Parameters der Druckluft werden ein Sensor zur Erfassung einer Konzentration an Kohlenstoffdioxid, ein Sensor zur Erfassung einer Konzentration an Stickstoffdioxid, ein Sensor zur Erfassung einer Konzentration an Kohlenstoffmonoxid, ein Sensor zur Erfassung einer Konzentration an Schwefeldioxid, ein Sensor zur Erfassung einer Konzentration an Sauerstoff sowie ein Sensor zur Erfassung einer relativen Luftfeuchtigkeit in der Messluftleitung genannt.

Die EP2148616B1 zeigt ein Messsystem mit einer Vielzahl von Sensorik wie Durchflusssensorik, Temperatursensorik, Drucksensorik, Feuchtesensorik, Gassensorik zur Messung von Sauerstoff, Kohlenstoffdioxid, Kohlenstoffmonoxid, Stickstoff, Stickoxiden, Anästhesiegasen, Gasbestandteilen in der Ausatmung sowie weiteren Gasen.

Die DE102006030242A1 zeigt ein konfigurierbares Messsystem mit einer Vielzahl von Gassensoren. Als Gassensoren können elektrochemische, infrarot-optische und katalytische Gassensoren in dem Messsystem konfiguriert werden. Aus der US20130167843A1 ist eine Pumpe zur Förderung von Luftmengen bekannt. Die Pumpe weist eine piezoelektrische Funktionsweise auf. Eine solche Pumpe eignet sich zu einer Förderung von Gasmengen von einem Messort zu einem Ort der Sensorik und/oder messtechnischen Erfassung mittels einer Messgasleitung (sample line) und ist beispielsweise geeignet für den Einsatz bei einer Nebenstrommessung (side stream) zu einer Analyse von Gasbestandteilen - insbesondere auch Kohlenstoffdioxid und Sauerstoff - nahe am Mund-/Nasen-Bereich einer Person oder Patienten zur Analyse der In- und/oder Ausatemluft. Verschiedenste Ausführungen und Ausgestaltungen von und mit Gasfördereinrichtungen, Pumpen oder Vorrichtungen zum Gastransport für die Versorgung von Personen mit Atemgasen, teilweise in Ausgestaltungen und mit Eignung für die Beatmung von Personen, sind aus den Patentdokumenten WO2018033224A1, US20180163712A1, WO2018033225A1, US20180133420A1, US20180110957A1, WO2019072606A1, DE102017009605A1, DE102017009606A1, DE102018004341A1 und auch DE202012013442U1 bekannt.

Weitere Ausgestaltungen von Gasfördereinrichtungen, Pumpen oder Vorrichtungen zum Gastransport für die Versorgung von Personen mit Atemgasen, teilweise in Ausgestaltungen und mit Eignung für die Beatmung von Personen sind aus der DE102019003643 A1, DE102019003607 A1, DE102019004450 A1 und DE102019004451A1 bekannt.

Verschiedenste Ausführungen und Ausgestaltungen von Gasfördereinrichtungen, Pumpen oder Vorrichtungen zum Gastransport für eine Zuführung von Messgasen zu einer Gasmessvorrichtung sind aus den Patentdokumenten DE102016013756A1, US20180143171A1, US20180143170A1, US20180335410A1 bekannt.

Auch in Anwendungen von Untersuchungsröhrchen zur Untersuchung von Umweltgasen, welche oftmals auch als Prüfröhrchen bezeichnet werden, sind Pumpen bekannt, die dazu angewendet werden, definierte Proben an Prüfgas zu einem solchen Prüfgas hin zu fördern. Diese können als einfach aufgebaute und manuell betriebene Handpumpen oder als automatische Pumpen ausgebildet sein. Auch die Anmelderin bietet manuell betriebene Handpumpen oder automatische Pumpen als Zubehör für Prüfröhrchen an.

Aus der US2008264418A ist ein Verbindungselement, ein sogenanntes Y-Stück, zu einem Anschluss von Beatmungsschläuchen am Mund-/Nasen-Bereich einer Person oder eines Patienten mit Sensorik-Komponenten, Komponenten zur Messwerterfassung, Signalverarbeitung, Signalauswertung und Anzeige bekannt. Die Sensorik-Komponenten umfassen Atemstromsensorik mit Drucksensorik, Sensorik zur Sauerstoffpartialdruckmessung, Temperatursensorik, Durchflussmengen-Sensorik, ausgestaltet als Hitzdrahtanemometer oder Ultraschalldurchflusssensor, sowie Anschlusselemente für EKG und Blutdruckmessung.

Aus der US7897109B, US7335164B, US6616896B, US5789660B und US6312389B sind Sauerstoffsensoren nach dem Messprinzip des sogenannten *luminescence quenching* bekannt, welche im Nebenstrom (side stream) im bzw. am Atemgasweg eines Patienten angeordnet werden können.

Aus der DE102010037923B4 ist ein Sauerstoffsensor mit einer Bioreaktor-Anordnung bekannt. Aus der US9867563B ist ein System zu einer Erkennung einer verminderten Sauerstoffversorgung bei Piloten und zu einer Reduzierung der Verminderung der Sauerstoffversorgung bei Piloten bekannt.

Aus der US2003194351A ist eine galvanische Zelle zur Messung von Sauerstoff bekannt. Aus der der DE102004062052B4, DE19726453C2 sind elektrochemische Sauerstoffsensoren bekannt. Aus der DE2155935 ist ein elektrochemischer Sensor zur Messung von gasförmigen Bestandteilen in einem Gasgemisch bekannt.

Aus der US5958200B, DE102009010773B4, DE102005026491B4, DE102005026306B4 und der US8496795B sind verschiedenste Ausgestaltungen von elektrochemischen Gassensoren bekannt, welche zu einer messtechnischen Erfassung von Sauerstoff oder anderen Gasen geeignet sind. Die DE102005007539A1 zeigt einen elektrochemischen Gassensor zu einer quantitativen Bestimmungen redoxaktiver Stoffe in sehr geringen Konzentrationsbereichen. Das elektrochemische Messprinzip eignet sich, je nach Ausgestaltung von Elektroden und Elektrolyt, zu einer Erfassung verschiedenster Gase, beispielsweise etwa Sauerstoff, Ammoniak, Schwefeldioxid, Wasserstoffperoxid, Schwefelwasserstoff, Stickstoffdioxid, Stickstoffmonoxid, Arsin, Silane, Formaldehyd, Acetylen, Kohlenstoffmonoxid, Phosgen, Phosphin. Aus der DE19912100A1 ist ein elektrochemischer Kohlenstoffmonoxidsensor bekannt.

Aus der US4851088B ist ein elektrochemischer Kohlenstoffdioxidsensor bekannt.

Die US5473304B und die DE4020385C2 zeigen in Keramikfolientechnik hergestellte Wärmetönungssensoren.

Die US7875244B, GB2210980A1 und die DE19610912A1 zeigen Wärmetönungssensoren in Pellistor-Ausführung.

Die US2010221148A, US5902556B zeigen katalytische Gassensoren mit Halbleiter-Chip als Messelemente. Aus der US2816863B, US2019178827A, US8425846B, US9625406B, US6756016B, US2016178412A, US6344174B sind katalytische Gassensoren bekannt. Das katalytische Messprinzip, auch als Prinzip der Wärmetönung bekannt, eignet sich besonders zu einer Erfassung von brennbaren und/oder explosiven Gasen, insbesondere Kohlenstoffwasserstoffverbindungen, wie auch zu einer Bestimmung von Restbestandteilen von Verbrennungsprozessen. Beispielsweise können Toluol, Ammoniak, Benzol, Propan, Methan, Methanol, Oktan, Butan, Ethylen mit dem Prinzip der Wärmetönung messtechnisch erfasst werden. Oftmals werden katalytische Sensoren zur Überwachung von Grenzwerten, etwa der UEG (Untere Explosionsgrenze), eingesetzt.

Die US4175422B zeigt einen Gassensor mit einem Halbleiter-Element als Messelement. Aus der US9958305B ist eine Gassensorvorrichtung mit Halbleiter-Sensorik in Ausgestaltung von Chip-Technologie zu einer Überwachung von Verbrennungsprozessen in Verbrennungsmotoren eines Kraftfahrzeuges bekannt.

Aus der DE102004048979B4, US4902138B sind miniaturisierte Halbleiter-Gassensoren bekannt. Aus der DE102012022136B4 ist ein Halbleiter-Kohlenstoffmonoxidsensor bekannt.

Aus der US9818937B und der US9234876B sind miniaturisierte Halbleiter-Sauerstoffsensoren, ausgeführt in mikrostrukturierter Technologie, sogenannter MEMS-Technologie, bekannt. Bekannt sind weiterhin Gassensoren mit Festkörper-Elektrolyten, etwa auf Basis von Zirkon-Dioxid.

So zeigt die DE102008056279B4 eine Anordnung mit einem beheizten Festelektrolyt-Sauerstoffsensor und einem Ultraschallsensor zur indirekten Erfassung der Konzentration von Kohlenstoffstoffdioxid.

Die US5026992B zeigt einen Gassensor zur messtechnisch-optischen
Erfassung von Methan.

Die US8399839B zeigt einen Gassensor zur messtechnisch-optischen Erfassung von Kohlenstoffdioxid.

Aus der EP0149619A1 ist eine Vorrichtung mit einer Lambdasonde zur Erfassung einer Menge an Restsauerstoff im Abgas eines Verbrennungsmotors bekannt.

Aus der US4667157B ist ein Hall-Effekt-Sauerstoffsensor bekannt.

Die US8596109B, US8596109B, US9360441B2, US4808921B, US6430987B, US6952947B, US6895802B, US6405578B, US4683426B, US4173975B, US3646803B, US3584499B, US2944418B und die WO16162287A1 zeigen Vorrichtungen zur Messung von Konzentrationen paramagnetischer Gase. Mit solchen Vorrichtungen ist insbesondere eine qualitative und auch quantitative messtechnische Erfassung von Sauerstoff möglich, da Sauerstoff paramagnetische Eigenschaften aufweist.

Aus der US9360441B ist ein Messelement für einen paramagnetischen Gassensor, insbesondere für einen Sauerstoffsensor, bekannt. Der paramagnetische Gassensor bzw. Sauerstoffsensor kann vorzugsweise im Nebenstrom (side stream) im bzw. am Atemgasweg eines Patienten angeordnet werden.

In der DE102010047159B4 und der US2004238746A sind Gasmessvorrichtungen beschrieben.

In der US5739535B ist eine infrarot-optische Gasmessvorrichtung beschrieben.

Aus der US8399839B ist ein infrarot-optischer Kohlenstoffdioxidsensor,
ein sogenannter IR-Kohlenstoffdioxidsensor, bekannt.

Aus der DE102010047159B4 und US6895802B sind Vorrichtungen zur Messung der Konzentration von Kohlenstoffdioxid in Atemgas durch Messung der Wärmeleitfähigkeit bekannt. Die Ausführung nach der DE102010047159B4 zeigt einen Kohlenstoffdioxidsensor mit einem Halbleiter-Chip als Messelement zur Erfassung von Wärmeleitfähigkeitsänderungen.

Aus der US5696379B, US2004203169A und der US4050823B sind infrarot-optische Kohlenstoffdioxidsensoren bekannt.

Aus der US8448642B, US5095900B, US5067492B, WO20109115A1, US2019105457A, US6095986B, USD727492S1 und der US5942755B sind infrarot-optische Kohlenstoffdioxidsensoren bekannt, welche im Hauptstrom (main stream) im Atemgasweg eines Patienten angeordnet werden können. Gasmessvorrichtungen oder Sensoren zur messtechnischen Erfassung von Kohlenstoffdioxid, insbesondere auch geeignet zur messtechnischen Erfassung von Kohlenstoffdioxid in Atemgasen, sind aus der US2002036266A, US2004238746A, US20180120224A1 und der US20180116555A1 bekannt. Weitere Gasmessvorrichtungen oder Sensoren zur messtechnischen Erfassung von Kohlenstoffdioxid sind aus der DE102020114972A1 und DE102020114968A1 bekannt.

Aus der US6571622B ist ein Kombinationssensor aus einem infrarot-optischen Kohlenstoffdioxidsensor mit einem Durchflusssensor bekannt, welcher im Hauptstrom (main stream) im Atemgasweg eines Patienten angeordnet werden kann. Aus der US2004238746A, US2002036266A sind infrarot-optische Kohlenstoffdioxidsensoren bekannt, welche im Nebenstrom (side stream) im bzw. am Atemgasweg eines Patienten angeordnet werden können.

Die US6954702B, US7606668B, US8080798B, US7501630B, US7684931B, US7432508B, US7183552B zeigen Gasmesssysteme zur Erfassung von Gaskonzentrationen im Nebenstrom (side stream) und Hauptstrom (main stream).

In der US9939374B und der US7705991B sind Interferometer in Ausgestaltungen von Gasmessvorrichtungen beschrieben.

Aus der US6274879B und EP2788739B1 sind laser-basierte Anordnungen zu einer Erfassung von Gasbestandteilen bekannt.

Aus der US9459235B ist ein Gassensor in einer Ausführung als Photoionisationsdetektor bekannt. Weitere Aspekte hinsichtlich einer qualitativen und quantitativen Zusammensetzung des Atemgases beziehen sich darauf, dass das Atemgas weitgehend frei von Verunreinigungen, beispielsweise weitgehend frei von Fremdkörpern oder Partikeln, sein sollte wie etwa Ruß, Staub, Pollen oder Ausdünstungen von Materialien, welche das Atemgas auf dem Weg zu Flugzeugführern, Piloten, Copiloten durchströmen.

Weiterhein sollten sich keine oder keine wesentlichen Mengen gesundheitlich bedenklicher Gase oder Gasgemische, wie etwa Kohlenstoffmonoxid (CO), Ozon, Spuren weiterer Gase oder Spuren von Flugbenzin oder Kerosin, Mengen an Abgasen oder Rückständen der Verbrennung oder weitere Luftschadstoffe, im Atemgas befinden. Dazu zählen beispielsweise verschiedenste Zusammensetzungen von Kohlenstoffwasserstoffen, Benzole, Stickoxide (NO₂, NOₓ), Schwefeloxide, (SO₂ SOₓ), Dioxine, Furane, Partikel, z.B. Ruß, Feinstaub, ultrafeine Partikel. Neben der o.g. Kohlenstoffdioxidvergiftung sei in dem Zusammenhang auch besonders auf die Kohlenstoffmonoxidvergiftung hingewiesen. Bereits Konzentrationen oberhalb von 200 ppm (0,02 %) verursachen Kopfschmerzen und einen Verlust des Urteilsvermögens, Konzentrationen oberhalb von 800 ppm (0,08 %) verursachen Schwindel, Unruhe, Übelkeit, Angst und Krämpfe innerhalb von 45 Minuten und Bewusstlosigkeit innerhalb von 2 Stunden, ggf. mit Todesfolge. Bei einer Kohlenstoffmonoxidvergiftung kommt es zu einer Herabsetzung der Sauerstoff-Transportkapazität des Bluts durch eine Verminderung des Hämoglobingehalts (Anämie) oder durch Beeinträchtigung des Sauerstoff-Bindungsvermögens im Blut, es kommt zu einer anämischen Hypoxie.

Es ergibt sich daher ein Bedarf, für Flugzeugführer, Piloten, Copiloten die Situation abzusichern, dass von der Bordtechnik eines Luftfahrzeuges oder Fluggerätes während des Flugbetriebes stets einwandfreies und qualitativ hochwertiges Atemgas bereitgestellt wird und an Flugzeugführer, Piloten, Copiloten von Luftfahrzeugen oder Fluggeräten verabreicht werden kann.

Daraus resultiert die Aufgabe der vorliegenden Erfindung, ein Überwachungssystem für Flugzeugführer, Piloten, Copiloten von Luftfahrzeugen oder Fluggeräten bereitzustellen, welches eine messtechnische Überwachung von Atemgasen bzw. Atemluft in Luftfahrzeugen oder Fluggeräten ermöglicht.

Ergänzt wird diese Aufgabe dadurch, dem Flugzeugführer, Piloten oder Copiloten an dem Überwachungssystem eine Eingabemöglichkeit bereitzustellen.

Die Aufgabe wird durch ein Überwachungssystem zu einer Überwachung einer Gaszusammensetzung von Atemgasen in Luftfahrzeugen oder Fluggeräten mit den Merkmalen des unabhängigen Anspruchs 1 gelöst.

Weitere Merkmale und Details der Erfindung und vorteilhafte Ausgestaltungen ergeben sich aus den Unteransprüchen, der Beschreibung und den Zeichnungen. Dabei verwendete Rückbeziehungen weisen auf die weitere Ausbildung des Gegenstandes des Hauptanspruches durch die Merkmale des jeweiligen Unteranspruches hin und sind nicht als ein Verzicht auf die Erzielung eines selbständigen, gegenständlichen Schutzes für die Merkmalskombinationen der rückbezogenen Unteransprüche zu verstehen. Jede Bezugnahme in der Beschreibung auf Aspekte nachgeordneter Ansprüche ist demnach auch ohne speziellen Hinweis ausdrücklich als Beschreibung optionaler Merkmale zu lesen. Ausführungsformen schaffen Möglichkeiten zu einer messtechnischen Überwachung der Gaszusammensetzung von Luft, Atemluft oder Atemgasen in Luftfahrzeugen oder Fluggeräten. Zumindest manche Ausführungsbeispiele der Erfindung beziehen sich auf ein Überwachungssystem zur Überwachung der Gaszusammensetzung von Luft, Atemluft oder Atemgasen in Luftfahrzeugen oder Fluggeräten.

In zumindest manchen Ausführungsbeispielen kann eine messtechnische Erfassung von Eigenschaften mindestens eines Gases mittels einer Sensorik eines Überwachungssystems ermöglicht sein.

Zu Eigenschaften eines Gases können beispielsweise physikalische und andere Eigenschaften zählen: Druck, Dichte, Viskosität, Wärmeleitfähigkeit, elektrische und magnetische Eigenschaften, Temperatur, Gaszusammensetzung, Feuchtegehalt, Toxizität, Brennwert, Brennbarkeit, Bindungsfähigkeiten mit anderen Gasen oder Flüssigkeiten beispielsweise Wasser oder Blut.

In zumindest manchen Ausführungsbeispielen kann eine qualitative messtechnische Erfassung mindestens eines Gases ermöglicht sein. In zumindest manchen Ausführungsbeispielen kann eine quantitative messtechnische Erfassung mindestens eines Gases und/oder einer Konzentration eines Gases ermöglicht sein. In zumindest manchen Ausführungsbeispielen kann eine qualitative und eine quantitative messtechnische Erfassung mindestens eines Gases ermöglicht sein. In zumindest manchen Ausführungsbeispielen kann eine qualitative und eine quantitative messtechnische Erfassung von Sauerstoff ermöglicht sein. In zumindest manchen Ausführungsbeispielen kann eine qualitative und eine quantitative messtechnische Erfassung von Kohlenstoffdioxid ermöglicht sein. In zumindest manchen Ausführungsbeispielen kann eine qualitative und eine quantitative messtechnische Erfassung eines weiteren Gases, insbesondere von Kohlenstoffmonoxid ermöglicht sein.

In zumindest manchen Ausführungsbeispielen ist eine Kontrolleinheit in dem Überwachungssystem angeordnet oder dem Überwachungssystem zugeordnet. Die Kontrolleinheit ist ausgebildet und dazu vorgesehen, einen Ablauf einer messtechnischen Überwachung der Gaszusammensetzung von Luft, Atemluft oder Atemgasen in Luftfahrzeugen oder Fluggeräten zu organisieren, zu kontrollieren, zu steuern oder zu regeln. Die Kontrolleinheit ist vorzugsweise aus Komponenten (µC, µP, PC) mit zugehörigem Betriebssystem (OS), Datenspeicher (RAM, ROM, EEPROM) sowie SW-Code, Software zur Ablaufsteuerung, Kontrolle, Steuerung, Regelung ausgebildet. In zumindest manchen Ausführungsbeispielen sind der Kontrolleinheit weitere ElektronikElemente wie beispielsweise Komponenten zu Signalerfassung (ADµC), Signalverstärkung, zu analoger und/oder digitaler Signalverarbeitung (ASIC), Komponenten zu analoger und/oder digitaler Signalfilterung (DSP, FPGA, GAL, µC, µP), Signalumwandlung (A/D-Wandler) zugeordnet oder mit der Kontrolleinheit verbunden.

In zumindest manchen Ausführungsbeispielen kann mittels der Sensorik eine qualitative und eine quantitative messtechnische Erfassung einer Konzentration von Sauerstoff ermöglicht sein. Die Konzentration von Sauerstoff kann dabei beispielsweise in Form eines Partialdrucks in einem Gasgemisch beispielsweise der Atemluft oder des Atemgases oder in Form einer Volumenkonzentration oder in Form einer Massenkonzentration messtechnisch ermittelt werden. In zumindest manchen Ausführungsbeispielen kann mittels der Sensorik eine qualitative und eine quantitative messtechnische Erfassung einer Konzentration von Kohlenstoffdioxid ermöglicht sein.

Die Konzentration von Kohlenstoffdioxid kann dabei beispielsweise in Form eines Partialdrucks in einem Gasgemisch, beispielsweise der Atemluft oder des Atemgases oder in Form einer Volumenkonzentration oder in Form einer Massenkonzentration, messtechnisch ermittelt werden. In zumindest manchen Ausführungsbeispielen kann mittels der Sensorik eine qualitative und eine quantitative messtechnische Erfassung einer Konzentration von Kohlenstoffmonoxid ermöglicht sein. Die Konzentration von Kohlenstoffmonoxid kann dabei beispielsweise in Form eines Partialdrucks in einem Gasgemisch, beispielsweise der Atemluft oder des Atemgases oder in Form einer Volumenkonzentration oder in Form einer Massenkonzentration, messtechnisch ermittelt werden.

In zumindest manchen Ausführungsbeispielen kann die Sensorik mindestens einen Sensor aufweisen.

Dabei ist der mindestens eine Sensor vorzugsweise als ein Sauerstoffsensor, ein Kohlenstoffdioxidsensor oder mindestens ein weiterer Gassensor, insbesondere Kohlenstoffmonoxidsensor ausgebildet.

In zumindest manchen Ausführungsbeispielen kann zur qualitativen und quantitativen messtechnischen Erfassung der Konzentration von Sauerstoff ein paramagnetischer Sauerstoffsensor oder ein Messmodul mit einem paramagnetischen Sauerstoffsensor Verwendung finden. In einer weiteren vorteilhaften Weise kann dabei ein elektrochemischer Sauerstoffsensor oder ein Messmodul mit einem elektrochemischen Sauerstoffsensor Verwendung finden. In einer weiteren vorteilhaften Weise kann dabei ein Sauerstoffsensor oder ein Messmodul mit einem Sauerstoffsensor Verwendung finden, welcher nach dem Prinzip des Lumineszenz-Quenching oder Fluoreszenz-Quenching funktioniert. In einer weiteren vorteilhaften Weise kann dabei ein Halbleiter-Sauerstoffsensor, vorzugsweise in Form eines sogenannten MEMS-Sauerstoffsensors oder ein Messmodul mit einem Halbleiter-Sauerstoffsensor bzw. mit einem MEMS-Sauerstoffsensor, Verwendung finden. In einer weiteren vorteilhaften Weise kann dabei ein elektrochemischer Sauerstoffsensor und/oder paramagnetischer Sauerstoffsensor oder ein Messmodul mit einem elektrochemischen Sauerstoffsensor und/oder einem paramagnetischen Sauerstoffsensor Verwendung finden. In einer weiteren vorteilhaften Weise kann dabei ein elektrochemischer Sauerstoffsensor und/oder Halbleiter-Sauerstoffsensor oder ein Messmodul mit einem elektrochemischen Sauerstoffsensor und/oder einem Halbleiter-Sauerstoffsensor Verwendung finden.

In einer weiteren vorteilhaften Weise kann dabei ein paramagnetischer Sauerstoffsensor und/oder Halbleiter-Sauerstoffsensor und/oder einem elektrochemischen Sauerstoffsensor oder ein Messmodul mit einem paramagnetischen Sauerstoffsensor und/oder einem Halbleiter-Sauerstoffsensor und/oder einem elektrochemischen Sauerstoffsensor Verwendung finden.

In einer weiter vorteilhaften Weise kann dabei ein paramagnetischer Sauerstoffsensor und/oder Halbleiter-Sauerstoffsensor oder ein Messmodul mit einem paramagnetischen Sauerstoffsensor und/oder einem Halbleiter-Sauerstoffsensor Verwendung finden. In zumindest manchen Ausführungsbeispielen kann zur qualitativen und quantitativen messtechnischen Erfassung der Konzentration von Kohlenstoffdioxid ein optischer Kohlenstoffdioxidsensor, vorzugsweise in Form eines infrarot-optischen sogenannten IR-Kohlenstoffdioxidsensors oder ein Messmodul mit einem optischen, vorzugsweise infrarot-optischen Kohlenstoffdioxidsensor, einem sogenannten IR-Sensor, Verwendung finden.

In einer weiteren vorteilhaften Weise kann dabei ein Halbleiter-Kohlenstoffdioxidsensor, vorzugsweise in Form eines sogenannten MEMS-Kohlenstoffdioxidsensors oder ein Messmodul mit einem Halbleiter-Kohlenstoffdioxidsensor bzw. mit einem MEMS-Kohlenstoffdioxidsensor, Verwendung finden.

In einer weiteren vorteilhaften Weise kann dabei ein Halbleiter-Kohlenstoffdioxidsensor, vorzugsweise in Form eines sogenannten MEMS-Kohlenstoffdioxidsensors und/oder ein dabei optischer Kohlenstoffdioxidsensor, vorzugsweise in Form eines infrarot-optischen sogenannten IR-Kohlenstoffdioxidsensors oder ein Messmodul mit einem Halbleiter-Kohlenstoffdioxidsensor bzw. MEMS-Kohlenstoffdioxidsensor und/oder einem optischen Kohlenstoffdioxidsensor bzw. IR-Kohlenstoffdioxidsensors, Verwendung finden.

Die Messmodule mit mindestens einem Sauerstoffsensor werden im Kontext der vorliegenden Erfindung auch als Sauerstoffmessmodule bezeichnet. Die Messmodule mit mindestens einem Kohlenstoffdioxidsensor werden im Kontext der vorliegenden Erfindung auch als Kohlenstoffdioxidmessmodule bezeichnet.

Die Sauerstoffmessmodule und/oder Kohlenstoffdioxidmessmodule können in manchen Ausführungsformen noch weitere Sensoren aufweisen bzw. den Sauerstoffmessmodulen und/oder Kohlenstoffdioxidmessmodulen können in manchen Ausführungsformen noch weitere Sensoren zugeordnet und/oder an den Modulen angeordnet sein.

In manchen Ausführungsformen können das Sauerstoffmessmodul und/oder das Kohlenstoffdioxidmessmodul ggf. mit weiteren Gassensoren und ggf. mit weiteren Sensoren zur messtechnischen Erfassung von Messgrößen oder Stoffgrößen, wie beispielsweise Druck, Umgebungsdruck, Atemwegsdruck, Maskendruck, Dichte, Temperatur, Wärmeleitfähigkeit, Wärmekapazität, Volumenstrom, Massenstrom, Durchflussmengen, Volumina, miteinander kombiniert ausgeführt sein und als ein Gasmessmodul, Messmodul oder als ein Modul zur Umgebungs- oder Umweltanalytik ausgebildet sein.

So kann ein Drucksensor in dem Überwachungssystem beispielsweise als Element des Sauerstoffmessmoduls oder des Kohlenstoffdioxidmessmoduls angeordnet sein, welcher zu einer Erfassung eines Druckniveaus in der Messgasleitung ausgebildet ist.

Zudem kann ein Strömungssensor oder Durchflusssensor in dem Überwachungssystem beispielsweise als Element des Sauerstoffmessmoduls oder des Kohlenstoffdioxidmessmoduls angeordnet sein, welcher zu einer Erfassung einer Durchflussmenge oder einer Strömung in der Messgasleitung ausgebildet ist.

Messwerte des Strömungssensors, Durchflusssensors sowie auch des Drucksensors können der Kontrolleinheit bereitgestellt werden.

Das Überwachungssystem weist Module wie Gasmessmodule, Messmodule, Module zur Umgebungs- oder Umweltanalytik auf.

Das Überwachungssystem weist in einer bevorzugten Ausführungsform mindestens ein Modul zum Gastransport auf. Das Modul zum Gastransport weist dazu eine Gasfördereinrichtung in Ausgestaltung einer Pumpe mit einem Gasanschluss auf, die ausgebildet ist, eine bestimmte Menge an Gas von einem von der Sensorik bzw. dem Sauerstoffmessmodul, Kohlenstoffdioxidmessmodul oder Gasmessmodul entfernt liegenden Messort zu dem Sauerstoffmessmodul, Kohlenstoffdioxidmessmodul oder Gasmessmodul, bzw. zu dem Sauerstoffsensor, Kohlenstoffdioxidsensor zu fördern, damit die messtechnische Erfassung der Sauerstoffkonzentration und/oder Kohlenstoffdioxidkonzentration ermöglicht ist.

Die Pumpe bzw. das Modul zum Gastransport ist derart ausgebildet, Mengen oder Teilmengen an Atemgas oder Atemluft von einem Messort, insbesondere der Atemmaske und/oder aus Kabine oder Cockpit anzusaugen und hin zu dem Überwachungssystem bzw. dem Sauerstoffmessmodul und/oder dem Kohlenstoffdioxidmessmodul bzw. hin zu der Sensorik, insbesondere dem Sauerstoffsensor und/oder dem Kohlenstoffdioxidsensor zu fördern.

Die Atemmaske kann beispielsweise als Teilmaske, Halbmaske oder Vollmaske oder als Kombination eines Schutzhelms mit einer Maske ausgebildet sein.

Zu einer weitgehenden oder gänzlichen Verhinderung von Rückströmungen oder zur Vermeidung ungewollter Strömungen oder Durchströmung können zusätzlich Ventile in der Zuströmung vor der Pumpe oder in der Abströmung hinter der Pumpe angeordnet sein. Das Modul zum Gastransport wird vorzugsweise mit Hilfe einer Messgasleitung (sample line) pneumatisch und/oder fluidisch mit dem Messort gasführend verbunden.

Zur Überwachung der Atemgasversorgung von Flugzeugführer, Piloten oder Copiloten wird vorzugsweise als Messort eine gasführende Komponente im Gesichtsbereich, d.h. nahe am Mund-/Nasen-Bereich des Flugzeugführers, Piloten oder Copiloten verwendet. Ein Ende der Messgasleitung ist vorzugsweise derart am Mund-/Nasen-Bereich, beispielsweise an der Atemmaske angeordnet, um eine Strömung von Gasmengen von dem Mund-/Nasen-Bereich hin zu dem Modul zum Gastransport des Überwachungssystems zu ermöglichen. Das andere Ende der Messgasleitung ist vorzugsweise mit einem Gasanschluss zu einer Zuströmung in das Modul zum Gastransport derart pneumatisch oder fluidisch verbunden, dass eine Förderung von Mengen oder Teilmengen an Atemgas beispielsweise mit einer Durchflussmenge im Bereich von 25 ml/min bis zu 250 ml/min mittels des Moduls zum Gastransport hin zu dem Sauerstoffmessmodul und/oder dem Kohlenstoffdioxidmessmodul ermöglicht ist.

Dazu ist das Modul zum Gastransport mit einem weiteren Gasanschluss zur Abströmung oder Förderung mit dem Sauerstoffmessmodul und/oder dem Kohlenstoffdioxidmessmodul pneumatisch und/oder fluidisch verbunden. Mit Hilfe des Strömungssensors oder Durchflusssensors kann die Kontrolleinheit das Modul zum Gastransport kontrollieren und die Mengen an zu förderndem oder zu saugendem Gas in der Messgasleitung kontrollieren, steuern, regeln oder einstellen.

Mit Hilfe des Drucksensors kann die Kontrolleinheit das Druckniveau in der Messgasleitung überwachen und mittels des Moduls zum Gastransport auch kontrollieren, steuern, regeln oder einstellen. Wird der Durchflusssensor als Druckdifferenzsensor (ΔP-Sensor) einer Differenzmessung zweier Druckmesspunkte über einer Strömungsblende ausgestaltet, so kann mit Erfassung eines der zwei Druckmesspunkte mit Bezug zur Umgebung mit diesem Sensor auch eine Druckmessung des Druckniveaus in der Messgasleitung ermöglicht sein.

In einer bevorzugten Ausführungsform kann das Modul zum Gastransport mit der Pumpe an einem Gaseinlass des Überwachungssystems angeordnet sein. In einer solchen beispielhaften Konstellation saugt das Modul zum Gastransport Mengen an Gas durch die Messgasleitung von der Atemmaske des Flugzeugführers hin in das Überwachungssystem und fördert diese Mengen an Gas dann zu und hindurch durch die Sensorik zur Gaskonzentrationsbestimmung. Nach Durchströmung der Sensorik gelangen die Mengen an Gas durch einen Gasauslass in die Umgebung.

In einer weiter bevorzugten Ausführungsform kann das Modul zum Gastransport mit der Pumpe an einem Gasauslass des Überwachungssystems angeordnet. In einer solchen Konstellation saugt das Modul zum Gastransport Mengen an Gas durch die Messgasleitung von der Atemmaske des Flugzeugführers hin in das Überwachungssystem durch die Sensorik zur Gaskonzentrationsbestimmung hindurch.

Nach Durchströmung der Pumpe gelangen die Mengen an Gas durch einen Gasauslass in die Umgebung. Mögliche Verunreinigungen durch die Pumpe können mit der Anordnung der Pumpe am Gasauslass nicht zur Sensorik gelangen. Über die pneumatische und/oder fluidische Verbindung kann über das Modul zum Gastransport, insbesondere die Pumpe, ein Transport von Mengen oder Teilmengen an Atemgas hin zu dem Sauerstoffmessmodul und/oder dem Kohlenstoffdioxidmessmodul bzw. hin zu dem Sauerstoffsensor und/oder dem Kohlenstoffdioxidsensor erfolgen, so dass eine messtechnische Erfassung von Konzentrationen von Sauerstoff und/oder Kohlenstoffdioxid ermöglicht ist.

Das Überwachungssystem ist in einer Bauform ausgestaltet, dass es in oder an der Kleidung des Flugzeugführers, Piloten oder Copiloten anbringbar ist. Die Messgasleitung weist eine entsprechende Länge auf, so dass eine solche Anbringung ermöglicht ist. Besonders vorteilhaft ist eine Unterbringung bzw. Anbringung des Überwachungssystems in einer Brusttasche, Beintasche oder Oberschenkeltasche eines Pilotenanzugs (Flieger-Overall). Das Modul zur Gasförderung ist derart ausgelegt und konstruiert, Gasmengen vom Messort hin zu dem bevorzugten Ort der Anbringung in Brusttasche, Beintasche oder Oberschenkeltasche des Pilotenanzugs fördern zu können. Das Modul zum Gastransport kann beispielsweise als eine Kreiselpumpe, Axialpumpe, Radialpumpe, Kolbenpumpe oder eine Membranpumpe ausgebildet sein. Besonders vorteilhaft für dem Einsatz in dem Überwachungssystem im mobilen und energieautarken Einsatz ist eine Pumpe mit geringem Energieverbrauch.

Eine piezo-elektrisch betriebene Pumpe, oftmals auch Piezopumpe genannt, ermöglicht beispielsweise einen energiesparenden Einsatz zur Gaskonzentrationsmessung in dem Überwachungssystem. Eine solche Pumpe wird beispielsweise von Murata Manufacturing Corp. of Kyoto, Japan als sogenannter "piezoelectric blower" oder "Microblower" mit den Bezeichnungen MZB1001T02 wie auch MZB 1001 angeboten. Diese Pumpen blockieren auch ohne eine elektrische Ansteuerung oder Aktivierung nicht die Durchströmung, daher ist es im Einsatz für das Überwachungssystem von Vorteil, für ein reproduzierbares Blockieren oder Freigeben der Strömung in und durch die Messgasleitung ein Ventil bereitzustellen, welches die Durchströmung in der Messgasleitung sicher, reproduzierbar und mit zwei Zuständen "Freigabe" und "Blockade" eindeutig sicherstellt.

Für die Ausführungsform mit der Pumpe am Gasauslass wie auch die Ausführungsform mit der Pumpe am Gaseinlass bietet sich ein Abschaltventil, ein sogenanntes "Flow-Lock-Valve", an, welches vorzugsweise am Gasauslass angeordnet sein kann.

Die Anordnung des Abschaltventils am Gasauslass des Überwachungssystems ermöglich eine Verwendung des im Innern des Überwachungssystems angeordneten Drucksensors für die Bestimmung des Drucks in der Atemmaske des Flugzeugführers mittels eines Messmanövers zur Bestimmung des Atemmaskendrucks, da bei geschlossenem Abschaltventil im strömungsfreien Zustand das Druckniveau im Innern des Überwachungssystems dem Druckniveau in der Messgasleitung wie auch dem Druckniveau in der Atemmaske entspricht.

Für die Ausführungsform mit der Pumpe am Gaseinlass bietet sich alternativ ein Umschaltventil, ein sogenanntes "3/2-Wege-Ventil", an, welches vorzugsweise am Gaseinlass angeordnet sein kann. Dies Umschaltventil ermöglicht einerseits eine Zuführung von Mengen an Gas aus der Messgasleitung in das Überwachungssystem, andererseits kann damit auch eine Zuführung von Gasmengen aus der Umgebung, d.h. der Kabine des Fluggerätes ermöglicht sein.

Während der Zuführung von Gasmengen aus der Kabine kann die Kontrolleinheit zeitgleich mittels eines Messmanövers zur Bestimmung des Atemmaskendrucks das Druckniveau in der Atemmaske bestimmen.

In einer bevorzugten Ausführungsform ist in dem Überwachungssystem ein weiterer Gasanschluss mit einem Umschaltventil angeordnet.

In einer weiter bevorzugten Ausführungsform ist in oder an dem Modul zum Gastransport ein weiterer Gasanschluss mit einem Umschaltventil angeordnet. Dieses Umschaltventil ermöglicht eine Umschaltung zwischen einer Zuführung von Mengen an Gas aus der Messgasleitung und einer Zuführung von Gasmengen mittels des weiteren Gasanschlusses aus der Umgebung, beispielsweise aus der der Kabine des Fluggerätes. In einer weiter bevorzugten Ausführungsform ist in oder an dem weiteren Gasanschluss eine weitere Pumpe angeordnet. Diese weitere Pumpe ermöglicht eine Zuführung von Gasmengen mittels des weiteren Gasanschlusses aus der Umgebung, beispielsweise aus der der Kabine des Fluggerätes.

Für die Ausführungsform mit der Pumpe am Gasauslass kann zusätzlich zum Abschaltventil am Gasauslass ein optionales Umschaltventil am Gaseinlass zu einer Umschaltung zwischen einer Überwachung von Gasmengen aus der Messgasleitung von der Atemmaske und von Gasmengen aus der Kabine angeordnet sein. Damit ist es dann der Kontrolleinheit ermöglicht, unabhängig von Zeitpunkten der Maskendruckbestimmung, jederzeit eine Umschaltung zwischen der Zuführung von Mengen an Atemgas von der Atemmaske des Flugzeugführers und einer Zuführung von Gas aus der Kabine durchführen zu können.

In manchen Ausführungsformen kann die Kontrolleinheit ausgebildet sein, aus den Messwerten des Kohlenstoffdioxidsensors eine Atemphaseninformation, also eine zeitliche Dauer einer Inspiration, eine zeitliche Dauer einer Exspiration, eine Verhältnis (I:E-ratio) von Inspirationszeitdauer zu Exspirationszeitdauer sowie eine Atemfrequenz des Luftfahrzeugführers, Piloten oder Copiloten zu bestimmen.

In zumindest manchen Ausführungsbeispielen können die Sensorik und die Kontrolleinheit zu einer Erfassung mindestens eines Umgebungsparameters und/oder mindestens eines Betriebsparameters ausgebildet sein. Betriebsparameter können beispielsweise Parameter aus dem Flugbetrieb, Parameter aus der Versorgung des Flugzeugführers, Piloten oder Copiloten mit Atemgasen, Parameter aus Kontrolle, Steuerung, Regelung des Fluggerätes oder von Komponenten des Fluggerätes sein.

In zumindest manchen Ausführungsbeispielen können die Sensorik und die Kontrolleinheit dazu ausgebildet sein, bei der Kontrolle des Ablaufs der messtechnischen Überwachung mindestens einen Umgebungsparameter mit zu berücksichtigen und/oder in den Ablauf mit einzubeziehen.

In einer bevorzugten Ausführungsform ist die Kontrolleinheit in Verbindung gemeinsam mit einem Drucksensor zu einer Bestimmung eines aktuellen Druckniveaus in der Atemmaske ausgebildet. Im Flugbetrieb eines Strahlflugzeugs (Jet) erfolgt die Versorgung der Flugzeugführer (Piloten, Copiloten) mit Hilfe einer am Mund-/Nasen-Bereich angeordneten Atemmaske. Von besonderem Interesse ist daher dabei die Überwachung des aktuellen Druckniveaus in der Atemmaske des Flugzeugführers, Piloten oder Copiloten. Damit kann sichergestellt werden, dass dem Flugzeugführer, Piloten oder Copiloten im Flugbetrieb ein ausreichendes Druckniveau an Atemgas mittels der Atemmaske bereitgestellt wird.

Ausführungsformen zeigen Möglichkeiten, mit Hilfe der Sensorik und der Kontrolleinheit eine Erfassung von Druckniveaus in der Atemmaske auszugestalten und somit das Druckniveau in der Atemmaske zu überwachen, bereitzustellen, auszugeben und/oder zu dokumentieren. Die Kontrolleinheit erfasst ein Druckniveau in der Messgasleitung mit Hilfe eines Drucksensors, welcher in dem Überwachungssystem angeordnet und in einem pneumatischen System mit den Komponenten Atemmaske, Messgasleitung, Anschlusselementen und einem optional in die Messgasleitung in Serienschaltung angeordneten HME-Filterelement pneumatisch und fluidisch verbunden ist, einen Druckmesswert zu erfassen, welcher ein Druckniveau in dem pneumatischen System indiziert. In einer weiter bevorzugten Ausführungsform zur Erfassung des aktuellen Druckniveaus wird in einer Messsituation im Betrieb des Überwachungssystems, in welchem bei deaktiviertem Modul zum Gastransport bzw. bei deaktivierter Pumpe keine Mengen an Gas von der Atemmaske der Sensorik zugeführt werden, also die Gaskonzentrationsmessung durch die Sensorik zeitweise unterbrochen ist bzw. pausiert, eine Druckmessung durch die Kontrolleinheit initiiert. In einer solchen Messsituation entspricht der Messwert, welcher das Druckniveau in dem pneumatischen System indiziert, dem aktuellen Druckniveau in der Atemmaske.

Zusätzlich zur Deaktivierung der Pumpe kann das Abschaltventil in einen geschlossenen Zustand versetzt werden, um jeglichen Gasaustausch des pneumatischen Systems mit der Umgebung zu unterbinden.

Mit einer solchen Ausführungsform kann diskontinuierlich eine Realisierung einer Messung und Überprüfung des Druckniveaus in der Atemmaske durchgeführt werden, wenn in bestimmten Zeitabständen eine Deaktivierung der Gasmengenzuführung durch die Pumpe erfolgt.

In einer weiter bevorzugten Ausführungsform zur Erfassung des aktuellen Druckniveaus wird im laufenden Betrieb des Überwachungssystems, in welchem mittels des Moduls zum Gastransport bzw. mittels aktivierter Pumpe fortlaufend Mengen an Gas von der Atemmaske der Sensorik zugeführt werden, eine Druckmessung durch die Kontrolleinheit initiiert. Mit einer solchen Ausführungsform kann kontinuierlich eine Realisierung einer Messung und Überprüfung des Druckniveaus in der Atemmaske durchgeführt werden, wenn diskontinuierlich zu bestimmten Zeitabständen eine Anpassung oder Kalibration an sich im Betrieb verändernde Druckabfälle des pneumatischen Systems mit den Komponenten Atemmaske, Messgasleitung, Anschlusselemente und dem HME-Filterelement erfolgt.

In einer solchen bevorzugten Ausführungsform kann eine diskontinuierlich und zu bestimmten Zeitabständen durchführbare Anpassung oder Kalibration durch ein Messmanöver ausgestaltet werden, welches von der Kontrolleinheit in Zusammenwirkung mit dem Modul zum Gastransport bzw. der Pumpe, dem Abschaltventil und einem Datenspeicher koordiniert und durchgeführt wird. Ein solches Messmanöver kann während des Flugbetriebes von Zeit zu Zeit durchgeführt werden, um Veränderungen in oder an dem pneumatischen System zu bestimmten Zeitpunkten im Zeitverlauf des Betriebes des Überwachungssystems während des Einsatzes am Flugzeugführer fortlaufend zu bestimmen. Das Messmanöver umfasst eine messtechnische Erfassung von Druckniveaus zu einer Nullung oder Offset-Ermittlung an zwei Arbeitspunkten.

Das Messmanöver gliedert sich in eine Druckmessung eines statischen Druckniveaus an einem Arbeitspunkt ohne eine Gasströmung im pneumatischen System und eine messtechnische Erfassung eines dynamischen Druckniveaus in Form einer Messung an einem weiteren vorgegebenen Arbeitspunkt mit einer definierten Strömung im pneumatischen System. Bei der messtechnischen Erfassung des statischen Druckniveaus wird ein Druckmesswert ohne eine Gasströmung innerhalb des pneumatischen Systems mit den Komponenten Atemmaske, Messgasleitung, Anschlusselementen und einem optional in die Messgasleitung in Serienschaltung angeordneten HME-Filterelement erfasst. Ohne eine Gasströmung, d.h. bei abgeschalteter Pumpe und einer resultierenden Strömungsmenge von 0,00 ml/min, treten keine durch Komponenten bedingte Druckabfälle im pneumatischen System zwischen der Atemmaske und dem Drucksensor bzw. der Pumpe im Überwachungssystem auf.

Das HME-Filterelement dient dazu, dass Feuchtigkeit aus der Atemgasversorgung mit Atemschlauch und Atemmaske, welche durch die Ausatmung des Flugzeugführers im Betrieb in die Messgasleitung eingebracht wird, nicht in das Überwachungssystem zu einer Überwachung einer Gaszusammensetzung von Atemgasen gelangen kann.

Ein solches HME-Filterelement (HME = Heat Moisture Exchange) ist ausgebildet, Mengen an Feuchtigkeit zurückzuhalten. Das HME-Filterelement ist in einer vorzugsweisen Ausführungsform in der Messgasleitung, am Gaseinlass oder am Modul zu Gastransport angeordnet. Im Betrieb reichern sich - bedingt durch die Ausatmung von feuchten Atemgasen durch den Flugzeugführer - fortlaufend Mengen an Feuchtigkeit oder Flüssigkeit im HME-Filterelement an. Daraus resultieren Veränderungen des Strömungswiderstandes über den Zeitverlauf der Einsatzdauer im Flugbetrieb mit Betrieb des Überwachungssystems. Neben der Abschaltung der Pumpe wird in vorteilhafter Weise das Abschaltventil in einen geschlossenen Zustand versetzt werden, um jeglichen Gasaustausch des pneumatischen Systems mit der Umgebung zu unterbinden.

Der erfasste Druckmesswert ohne Gasströmung in der Messgasleitung entspricht einer Momentaufnahme des aktuellen Drucks in der Atemmaske bei geschlossenem Abschaltventil und wird als ein statischer Druck des pneumatischen Systems in einem Datenspeicher abgelegt. Bei der messtechnischen Erfassung des dynamischen Druckniveaus wird ein Druckmesswert mit einer definierten Menge einer Gasströmung mit dieser Gasströmung entsprechenden Druckabfällen an den Komponenten des pneumatischen Systems mit Messgasleitung, Anschlusselementen und dem optionalen HME-Filterelement erfasst. Der erfasste Druckmesswert mit definierter Menge einer Gasströmung wird als ein dynamischer Druck des pneumatischen Systems im Datenspeicher abgelegt. Als geeignete und definierte Menge der Gasströmung in der Messgasleitung kann ein Bereich von 10 ml/min bis zu 400 ml/min von der Kontrolleinheit aktiviert, kontrolliert, gesteuert oder eingeregelt werden.

Der Druckmesswert mit Strömung entspricht dem dynamischen aktuellen gesamten Druckabfall des pneumatischen Systems. Dieser Druckmesswert entspricht dann der Summe der Druckabfälle im pneumatischen System, d.h. mit Druckabfällen über den Komponenten wie Atemmaske, HME-Filterelement, Messgasleitung und Verbindungselementen. Die Kontrolleinheit kann aus der Differenz des zuvor ermittelten statischen Druckniveaus und der Summe der dynamischen Druckabfälle den auf die Komponenten zurückgehenden Druckabfall als ein Offsetdruckniveau im pneumatischen System bestimmen.

Veränderungen der ermittelten Differenzen zwischen dem dynamischen und dem statischen Druckmesswerten zwischen zwei oder mehreren Zeitpunkten der Durchführung des Messmanövers ermöglichen es der Kontrolleinheit, Rückschlüsse in Bezug auf Veränderungen der Druckabfälle und Veränderungen des Offsetdruckniveaus im pneumatischen System während des Betriebes zu ziehen.

Diese Offsetdruckniveaus im pneumatischen System und deren Differenzen sowie deren Veränderungen werden fortlaufend während des Flugbetriebes durch die Kontrolleinheit erfasst bzw. bestimmt und im Datenspeicher - beispielweise in Form eines Datensatzes oder einer Tabelle oder als ein Log-File - abgelegt. Die Kontrolleinheit ist mittels des Messmanövers in dieser bevorzugten Ausführungsform vorteilhaft ausgebildet, durch eine messtechnische Erfassung statischer und dynamischer Druckabfälle über dem pneumatischen System bestimmte Offsetdruckniveaus als Kalibrierwerte für eine Bestimmung des aktuellen Maskendrucks zu bestimmen, anschließend bereitzustellen, auszugeben und/oder in Form von Datensätzen oder Tabellen zu speichern. Das Messmanöver liefert damit Trends und Veränderungen des Offsetdruckniveaus im Betrieb des Überwachungssystems am Flugzeugführer, Piloten oder Copiloten im Einsatz.

Auf diese Weise ist es möglich, auch unter sich während des Flugbetriebs verändernden Komponenten des pneumatischen Systems den jeweils aktuell gegebenen Maskendruck des Flugzeugführers zu bestimmen und zu überwachen. Insbesondere kann durch fortlaufende Wiederholungen des Messmanövers ein durch Feuchtigkeits-Aufsättigung bedingter Anstieg des Druckabfalls über dem HME-Filterelement als Veränderung des Offsetdruckniveaus erfasst und bei der Berechnung aktueller Druckniveaus in der Atemmaske ausgeglichen werden. Solche Wiederholungen der Überprüfung können beispielsweise alle 15 bis 60 Minuten stattfinden, eine häufigere Durchführung ist nicht vorteilhaft, da für die Durchführung des Manövers die Überwachung hinsichtlich der Gaskonzentrationen kurzzeitig ausgesetzt ist bzw. pausiert.

Die Bestimmung des aktuellen Drucks in der Atemmaske kann auf Basis einer Verwendung des mit dem Messmanöver letztmalig ermittelten Offsetdruckniveaus der Komponenten des pneumatischen Systems durch die Kontrolleinheit auch während des Betriebes mit aktivierter Pumpe und messtechnischer Erfassung der Gaskonzentrationen von Sauerstoff und/oder Kohlenstoffdioxid wie auch ggf. weiterer Gase oder der Kabinenluft durchgeführt werden.

Das aktuelle in der Atemmaske gegebene Druckniveau ergibt sich, indem vom aktuellen Druckmesswert bei Durchströmung der Messgasleitung, der das Druckniveau im pneumatischen System indiziert, das im Datenspeicher abgelegte und dort bereitgestellte letztmalig ermittelte Offsetdruckniveau subtrahiert wird.

Nachfolgend wird das Messmanöver, welches zur Bestimmung des Drucks in der Atemmaske bereits zuvor beschrieben wurde, auch im Hinblick auf die Einbindung dieses Messmanövers in den Messbetrieb des Überwachungssystems zur messtechnischen Erfassung der Gaskonzentrationen, vorzugsweise von Kohlenstoffdioxid und Sauerstoff, mit den Funktionen der dabei beteiligten Komponenten erläutert.

Das Messmanöver kann zu vorbestimmten Zeitpunkten aus dem laufenden Messbetrieb des Überwachungssystems aktiviert oder gestartet werden.

Folgende Schritte werden in einer Schrittabfolge von einem Start bis zu einem Ende von der Kontrolleinheit aktiviert, initiiert und durchgeführt:
- in einem ersten Schritt wird eine Deaktivierung der Pumpe vorgenommen,
- in einem zweiten Schritt erfolgt ein Verschließen des Abschaltventils,
- in einem dritten Schritt wird ein erster Messvorgang durch den Drucksensor mit einer Druckmessung zur Bestimmung des statischen Druckniveaus ausgeführt,
- in einem vierten Schritt erfolgt eine Öffnung des Abschaltventils,
- in einem fünften Schritt erfolgt eine Aktivierung der Pumpe zu einer Förderung einer definierten Fördermenge im Bereich von 50 ml/min bis 100 ml/min Mengen an Gas von der Atemmaske durch die Messgasleitung in das Überwachungssystem zu der Sensorik; dabei erfolgt eine Kontrolle und Überwachung der Fördermenge durch eine Durchflussmessung mittels des Durchflusssensors,
- in einem sechsten Schritt wird ein weiterer Messvorgang durch den Drucksensor, d. h. eine Druckmessung zur Bestimmung des dynamischen Druckniveaus ausgeführt,
- in einem siebten Schritt wird mit den Druckmesswerten der ersten Druckmessung und der weiteren Druckmessung eine Ermittlung eines Differenzwertes durchgeführt.

Der so ermittelte Differenzwert stellt das Offsetdruckniveau dar und kann für die Bestimmung eines Maskendrucks im weiteren Betrieb des Überwachungssystems im Einsatz des Fluggerätes als Kalibrierwert bereitgestellt und verwendet werden.

In optionalen Ausgestaltungen der Schrittabfolge des Messmanövers können von der Kontrolleinheit im dritten, fünften und sechsten Schritt die messtechnischen Erfassungen der statischen und dynamischen Druckniveaus und/oder der Durchflussmengen synchronisiert mit der Atmung des Flugzeugführers, Piloten oder Copiloten vorgenommen werden. So können die Druckmessungen und/oder die Durchflussmessungen vorzugsweise während inspiratorischer oder exspiratorischer Pausen ausgeführt werden.

In einer bevorzugten Ausführungsform kann die Kontrolleinheit ausgebildet sein, bei der Durchführung des Messmanövers zur Bestimmung des Drucks in der Atemmaske Informationen hinsichtlich Atemphasen des Flugzeugführers bei der messtechnischen Erfassung und/oder Bestimmung des statischen Druckmesswertes und/oder des dynamischen Druckmesswertes mit zu berücksichtigen. Die Erfassung der Druckmesswerte mit einer Synchronisation mit der Atmung mit Durchführung der Messwerterfassung in Pausen zwischen Ein- und Ausatmung ist vorteilhaft, da in solchen Pausen keine dem statischen und/oder dynamischen Druckniveaus durch die Atmung bedingte überlagerte Druckeffekte die Druckmesswerte verfremden oder beeinflussen können. Die Synchronisation mit der Atmung kann von der Kontrolleinheit mit Hilfe von Atemphaseninformationen basierend auf im Überwachungssystem messtechnisch erfassten Konzentrationsveränderungen an Kohlenstoffdioxid und/oder Sauerstoff durchgeführt werden. Die physiologischen Konzentrationsunterschiede im Sauerstoffgehalt des Atemgases zwischen Einatmung (21%) und Ausatmung (16%) wie auch Konzentrationsunterschiede im Kohlenstoffdioxidgehalt zwischen Ausatmung (~5%) und Einatmung (<1%) können von der Kontrolleinheit zur Ermittlung von Atemphasen genutzt werden. Ohne eine derartige Synchronisation der Druckmessung ist für die Signalverarbeitung eine geeignete Signalfilterung, beispielsweise mittels Tiefpass oder eine - vorzugsweise gleitende - Mittelwertbildung der Messwerte des Drucksensors, sinnvoll, um die Anteile der Atmung oder der Atemfrequenz aus den Druckmesswerten zu entfernen.

Daher kann in einer besonders bevorzugten Ausführungsform vorgesehen sein, dass die Kontrolleinheit zusammen mit der Signalverarbeitung unter Verwendung geeigneter Signalfilterung ausgebildet ist, die statischen und/oder dynamischen Druckmesswerte unter Entfernung von durch die Atmung des Flugzeugführers induzierten Signalanteilen mittels Signalfilterung zu bestimmen. In vorteilhafter Weise kann unter Verwendung des Umschaltventils während der Zeit der Maskendruckbestimmung eine Gasanalyse der Kabinenluft erfolgen. Von einem externen System können Vorgabewerte (Sollwerte), Bezugswerte als Schwellenwerte des Atemmaskendrucks, beispielsweise über eine Datenschnittstelle, bereitgestellt werden. Auf Basis solcher Werte kann das Überwachungssystem dann eine Alarmierungssituation bei Über- oder Unterschreitungen der Schwellenwerte bestimmen und entsprechende Alarmsignale und/oder Daten bereitstellen. Eine solche Bereitstellung kann beispielsweise drahtgebunden, drahtlos mittels Funkübertragung, drahtlos mittels Infrarot-Übertragung an externe Systeme erfolgen.

Weitere Möglichkeiten zur Alarmgabe an den Flugzeugführer werden durch visuelle, optische oder akustische Signalgabesysteme ermöglicht, etwa Lampen, Leuchtdioden, Anzeigeeinheiten, Lautsprecher, Summer, Hupen oder vergleichbare Elemente. Eine weitere Möglichkeit zur Alarmierung an den Flugzeugführer kann taktil, beispielsweise in Form eines Vibrationsalarms gegeben sein.

Weitere Ausführungsformen können zeigen, wie zusätzlich zum Maskendruck weitere Umgebungsparameter von der Kontrolleinheit bestimmbar sein können. Zu Umgebungsparametern während des Betriebes von Luftfahrzeugen oder Fluggeräten zählen beispielsweise:
- Umgebungsdruck außerhalb von Cockpit oder Kabine
   des Luftfahrzeugs oder Fluggerätes
- Umgebungstemperatur innerhalb von Cockpit oder Kabine des Luftfahrzeugs oder Fluggerätes
- Gaszusammensetzung innerhalb von Cockpit oder Kabine
   des Luftfahrzeugs oder Fluggerätes
- Absolute und/oder relative Feuchte innerhalb von Cockpit oder Kabine des Luftfahrzeugs oder Fluggerätes
- Dichte und/oder Umgebungsdruck innerhalb von Cockpit oder Kabine
   des Luftfahrzeugs oder Fluggerätes
- Umgebungstemperatur innerhalb von Cockpit oder Kabine
   des Luftfahrzeugs oder Fluggerätes
- Gaszusammensetzung innerhalb von Cockpit oder Kabine
   des Luftfahrzeugs oder Fluggerätes
- Umgebungsdruck außerhalb von Cockpit oder Kabine
   des Luftfahrzeugs oder Fluggerätes
- Umgebungstemperatur außerhalb von Cockpit oder Kabine
   des Luftfahrzeugs oder Fluggerätes
- Gaszusammensetzung außerhalb von Cockpit oder Kabine
   des Luftfahrzeugs oder Fluggerätes
- Absolute und/oder relative Feuchte außerhalb von Cockpit oder Kabine des Luftfahrzeugs oder Fluggerätes
- Dichte und/oder Umgebungsdruck außerhalb von Cockpit oder Kabine
   des Luftfahrzeugs oder Fluggerätes
- Umgebungstemperatur außerhalb von Cockpit oder Kabine des
   Luftfahrzeugs oder Fluggerätes
- Gaszusammensetzung außerhalb von Cockpit oder Kabine
   des Luftfahrzeugs oder Fluggerätes
- Druckniveau, Druckverlauf, Druck-Zeitverlauf,
   Druckunterschiede, Druckschwankungen in Atemgas, Atemgasgemisch oder in der Atemluft in der Zuführung zu Flugzeugführer, Pilot oder Copilot,
- Druckniveau, Druckverlauf, Druckunterschiede, Druckschwankungen in der Bereitstellung der Bordtechnik (z.B.: Gastanks, Drucksauerstoffflaschen, Luftansaugung, Gasaufbereitung, Filterung) für Atemgas, Atemgasgemisch oder Atemluft

In zumindest manchen Ausführungsbeispielen kann die Kontrolleinheit dazu ausgebildet sein, bei der Kontrolle des Ablaufs der messtechnischen Überwachung mindestens einen situativen Parameter mit zu berücksichtigen und/oder in den Ablauf mit einzubeziehen. Unter situativen bzw. aktuellen situativen Parametern sind Situationen und/oder sich aus Situationen ergebende Zustände während des Betriebes von Luftfahrzeugen oder Fluggeräten zu verstehen.

Dazu zählen beispielsweise:
- eine Flugrichtung
- eine Flughöhe
- eine Flugachsenlage
- eine Fluglage,
   beispielsweise etwa Rückenflug, Kurvenflug, Sturzflug, Sinkflug, Steigflug
- eine Fluggeschwindigkeit
- eine Flugrichtung
- eine horizontale Beschleunigung
- eine vertikale Beschleunigung
- ein Gierwinkel oder ein Rollwinkel
- ein Restvorrat an Sauerstoff oder Luft
- ein Restvorrat an Sauerstoffdruckgas oder Druckluft

In manchen Ausführungsformen kann das Überwachungssystem eine Datenschnittstelle aufweisen. Die Datenschnittstelle kann als unidirektionale oder bidirektionale Datenschnittstelle ausgebildet sein und beispielsweise zu Datenbereitstellung, Datenempfang, Datenaustausch oder Kommunikation mit Komponenten des Luftfahrzeuges oder Fluggerätes ausgebildet sein.

In zumindest manchen Ausführungsbeispielen können die situativen Parameter und/oder die Umgebungsparameter dem Überwachungssystem und/oder der Kontrolleinheit mittels der Datenschnittstelle empfangen und/oder bereitgestellt werden. In zumindest manchen Ausführungsbeispielen können die situativen Parameter und/oder die Umgebungsparameter mit Hilfe von in oder an dem Überwachungssystem angeordneten weiteren Sensoren der Sensorik messtechnisch erfasst werden und der Kontrolleinheit bereitgestellt werden. Dazu können in der Sensorik, neben der Sensorik zur messtechnischen Erfassung von Sauerstoff und/oder Kohlenstoffdioxid, weitere Gassensoren, beispielsweise zur messtechnischen Erfassung von Kohlenstoffmonoxid wie auch weitere Gassensoren, etwa in Form von elektrochemischen Gassensoren, katalytischen Gassensoren, optischen, infrarot-optischen Gassensoren, Photo-Ionisations-Gassensoren, Festkörperelektrolyt-Gassensoren oder Halbleiter-Gassensoren zum Einsatz kommen, um das Atemgas zusätzlich zur messtechnischen Erfassung von Konzentrationen an Sauerstoff und Kohlenstoffdioxid auch noch hinsichtlich weiterer Substanzen wie etwa Kohlenstoffwasserstoffe, Rückstände oder Produkte von Verbrennungsprozessen überwachen zu können. Als weitere Sensoren in der Sensorik können auch Drucksensoren vorgesehen sein, welche dazu ausgebildet sein können, einen Umgebungsdruck aus der Umgebung, insbesondere einen Druck oder eine Dichte innerhalb und/oder außerhalb von Cockpit oder Kabine des Luftfahrzeugs oder Fluggerätes messtechnisch zu erfassen und der Kontrolleinheit bereitzustellen. Die weiteren Sensoren in der Sensorik können als Temperatursensoren ausgestaltet sein, welche dazu ausgebildet und dazu vorgesehen sein können, eine Umgebungstemperatur der Umgebung, insbesondere eine Temperatur innerhalb und/oder außerhalb von Cockpit oder Kabine des Luftfahrzeugs oder Fluggerätes, messtechnisch zu erfassen und der Kontrolleinheit bereitzustellen. Diese weiteren Sensoren in der Sensorik können als Feuchtesensoren zu einer Erfassung einer absoluten oder relativen Feuchte der Umgebung ausgestaltet sein, welche dazu ausgebildet und dazu vorgesehen sein können, eine Feuchtigkeit in der Umgebung, insbesondere innerhalb und/oder außerhalb von Cockpit oder Kabine des Luftfahrzeugs oder Fluggerätes messtechnisch zu erfassen und der Kontrolleinheit bereitzustellen.

In manchen Ausführungsformen können weitere Sensoren an/in der Sensorik in dem Überwachungssystem zu einer Erfassung von Daten zur Ermittlung der situativen Parameter vorgesehen oder der Sensorik zugeordnet sein, welche der Kontrolleinheit eine Ermittlung einer eine aktuellen Flugsituation mit Flughöhe, Flugrichtung, Fluggeschwindigkeit, Flugbeschleunigung, Fluglage mit Ausrichtung im Raum und Flugsituation bzw. Flugmanöver (z.B. Steigen, Sinken, Kurvenflug, Landeanflug, Start) ermöglichen.

Dazu sind beispielsweise Drucksensoren, Beschleunigungssensoren, Höhensensoren, Kompass-Sensoren, Gyrosensoren, Feuchtesensoren, Temperatursensoren an oder in der Sensorik angeordnet oder der Sensorik zugeordnet.

In manchen Ausführungsformen kann die Sensorik sehr nahe am Mund-/Nasenbereich in oder an der Atemmaske angeordnet sein. Je nach strömungstechnischen Gegebenheiten am Mund-/Nasenbereich kann in solchen Fällen teilweise ein aktiver Transport von Atemgasen zu der Sensorik entfallen. Die Atemgase gelangen passiv, d.h. durch Diffusion aus dem Mund-/Nasenbereich in der Maske hin zu der Sensorik. In besonderen Ausgestaltungen kann eine Integration der Sensorik in die Atemmaske bzw. in oder an Teile der Atemmaske ermöglicht sein.

Durch die fortschreitende Technologieentwicklung im Bereich der Chip- und/oder MEMS-Technologie kann in naher Zukunft eine Miniaturisierung von Elementen der elektrochemischen, katalytischen oder Halbleiter-Sensorik erwartet werden, welche dann eine Integration der Sensorik, vorzugsweise mit Sauerstoff-Sensorik, Kohlenstoffdioxid-Sensorik und weiteren Gassensoren wie auch zusätzlicher und optionaler Drucksensorik und/oder Temperatursensorik direkt am Messort ermöglichen kann.

In manchen Ausführungsformen kann an dem Modul zum Gastransport ein weiterer Gasanschluss vorgesehen sein. Der weitere Gasanschluss ermöglicht einen Anschluss und eine Zuführung von Mengen oder Teilmengen an Gas oder Umgebungsluft aus der Kabine oder dem Cockpit hin zu dem Überwachungssystem.

Über ein Umschaltventil (z.B. 3/2-Wege-Ventil) oder ein System von Ventilen kann der Pumpe bzw. dem Modul zum Gastransport wahlweise Gas, Mengen oder Teilmengen aus der Umgebungsluft oder vom Mund-/Nasen-Bereich des Flugzeugführers, Piloten oder Copiloten beispielsweise von der Atemmaske zugeführt werden.

In manchen Ausführungsformen kann eine weitere Pumpe vorgesehen und derart angeordnet sein, dass eine Pumpe zu einem Transport von Gas, Mengen oder Teilmengen aus der Umgebungsluft hin zu dem Sauerstoffmessmodul und/oder dem Kohlenstoffdioxidmessmodul bzw. hin zu dem Sauerstoffsensor und/oder dem Kohlenstoffdioxidsensor vorgesehen und angeordnet ist und diese weitere Pumpe zu einem Transport von Gas, Mengen oder Teilmengen vom Mund-/Nasen-Bereich des Flugzeugführers, Piloten oder Copiloten, beispielsweise von der Atemmaske, hin zu dem Sauerstoffmessmodul und/oder dem Kohlenstoffdioxidmessmodul bzw. hin zu dem Sauerstoffsensor und/oder dem Kohlenstoffdioxidsensor vorgesehen und angeordnet ist. Damit kann in solch einer Ausführungsform ein Umschaltventil (z.B. 3/2-Wege-Ventil) oder ein System von Ventilen zur Umschaltung zwischen Mengen oder Teilmengen an Atemgas entfallen.

In manchen Ausführungsformen kann die Kontrolleinheit ausgebildet sein, das Modul zum Gastransport zu kontrollieren. Eine Kontrolle des Moduls zum Gastransport kann dabei eine Aktivierung, eine Deaktivierung, ein Einstellen, Steuern oder Regeln des Moduls zum Gastransport umfassen. Das Einstellen kann insbesondere eine Einstellung von Drehzahl, Fördermenge und/oder Druckniveau, beispielsweise mittels optischer oder elektrischer Steuersignale (CAN-Bus, PWM) oder elektrischer Steuerspannungen, umfassen.

In einer Variante solcher Ausführungsformen kann auf Basis der Erfassung von Gaskonzentrationsmesswerten und/oder Druckmesswerten - beispielsweise auch auf Basis von Druckdifferenzen zwischen Maske und Cockpit - eine Bestimmung hinsichtlich einer in der Messgasleitung vorhandenen Leckage erfolgen.

In manchen Ausführungsformen kann die Kontrolleinheit ferner ausgebildet sein, bei der Kontrolle des Moduls zum Gastransport mindestens einen Umgebungsparameter oder mindestens einen situativen Parameter mit zu berücksichtigen und/oder in die Kontrolle mit einzubeziehen.

Eine solche Berücksichtigung kann insbesondere eine Anpassung von Aktivierung, Deaktivierung, Drehzahl, Fördermenge und/oder Druckniveau des Moduls zur Gasförderung umfassen. Damit kann es ermöglicht sein, in bestimmten Flugmanövern, beispielsweise während eines Steigfluges, Sinkflug, oder Kurvenfluges, das Modul zum Gastransport zu deaktivieren und/oder nach Beendigung des Manövers ggf. mit erhöhter Fördermenge zu aktivieren.

In zumindest manchen Ausführungsbeispielen kann das Überwachungssystem und/oder die Kontrolleinheit zu einer Bestimmung und/oder Erkennung einer Alarmsituation und zu einer Organisation einer Alarmierung oder Alarmgabe und/oder zu einer Bereitstellung eines Alarmsignals ausgebildet sein. Die Kontrolleinheit kann anhand von Messwerten der Sensorik und/oder mittels der Datenschnittstelle bereitgestellter Informationen eine Alarmsituation bestimmen und/oder erkennen und eine Alarmierung auslösen und/oder ein Alarmsignal beispielsweise an der Datenschnittstelle oder einer anderen Datenschnittstelle bereitstellen. Die Alarmierung kann als eine visuelle und/oder eine akustische und/oder eine taktile Alarmierung erfolgen. Eine visuelle Alarmierung kann beispielsweise in Form eines weißen und/oder farbigen Leuchtmittels (LED, Stroboskop) oder einer Textausgabe (LCD, LED, Display) erfolgen.

Eine solche Alarmierung kann auf visuelle Weise auch mittels einer geeigneten Vorrichtung zur Visualisierung an oder in einer Gesichtsmaske oder Atemmaske erfolgen, beispielsweise als Darstellung auf einem In-Mask-Display oder Head-Up-Display. Eine akustische Alarmierung kann beispielsweise in Form einer Sprachausgabe oder mit Hilfe eines akustischen Alarmgebers (Hupe, Sirene) erfolgen. Eine taktile Alarmierung kann beispielsweise in Form eines Vibrations-Alarms an Ausrüstung des Fluggerätes, etwa Sitzflächen, Steuerungselementen (Fußpedale, Handgriffe) wie auch Ausrüstungsgegenständen (Atemmaske, Atemschlauch) oder Kleidung (Anzug, Weste, Fallschirm, Schuhe) des Luftfahrzeugführers, Piloten oder Copiloten, erfolgen.

In manchen Ausführungsformen kann die Kontrolleinheit bei der Organisation der Alarmierung oder Alarmgabe und/oder bei der Bereitstellung des Alarmsignals einen Umgebungsparameter und/oder einen situativen Parameter mit berücksichtigen und/oder mit in die Organisation der Alarmierung einbeziehen. Damit kann in vorteilhafter Weise ermöglicht werden, dass dem Luftfahrzeugführer, Piloten oder Copiloten in einer konsolidierten oder kompakten Art und Weise relevante und nach Relevanz priorisierte Alarm-Informationen hinsichtlich der Situation der messtechnischen Erfassung im Atemgas mit Bezug zur Situation in der Umgebung (Temperatur, Gaszusammensetzung in der Außenluft) und der Einsatzsituation bzw. Manöversituation des Luftfahrzeugs (Startphase, Landeanflug, Betankung in der Luft, Sinken, Kurvenflug, Steigen) gegeben werden können. In einer besonderen Ausgestaltung kann die Kontrolleinheit bei Durchführung der Signalverarbeitung und/oder Signalfilterung der Messwerte der Sensorik einen Umgebungsparameter und/oder einen situativen Parameter mit berücksichtigen und/oder mit in eine Anpassung der Signalverarbeitung einbeziehen.

In manchen Ausführungsformen kann die Kontrolleinheit bei der Organisation der Alarmierung vorbestimmte Schwellenwerte verwenden, welche für bestimmte Werte von Gaskonzentrationen, insbesondere Konzentrationen an Sauerstoff oder Kohlenstoffdioxid oder Kohlenstoffmonoxid, im Datenspeicher des Überwachungssystems hinterlegt sein können.

In manchen Ausführungsformen kann die Kontrolleinheit auf Basis von aktuellen und zeitlich zurückliegenden Konzentrationsmesswerten in Form einer Trendüberwachung von Sauerstoff und Kohlenstoffdioxid und mit Hilfe einer geeigneten Entscheidungsmatrix oder an das Problem speziell angepasster Algorithmen, lernfähigen oder selbstlernenden Algorithmen (SVM, Random Forest, AI, Deep-Learning, PCA), eine Art Frühwarnsystem zur Hypoxieerkennung ggf. mit daran angepasstem Alarmmanagement für den Beginn einer auftretenden Hypoxie zur Anwendung bringen. In einer besonderen Ausgestaltung kann die Kontrolleinheit dabei einen Umgebungsparameter und/oder einen situativen Parameter mit berücksichtigen. In einer besonderen Ausgestaltung kann die Kontrolleinheit dabei weitere beispielsweise mittels der Datenschnittstelle oder von dem Überwachungssystem zugeordneten Messsystemen bereitgestellte physiologische Daten von Luftfahrzeugführern, Flugzeugführern, Piloten, Copiloten, beispielsweise etwa EKG, Herzrate, Variabilität der Herzrate, Sauerstoffsättigung im Blut, Körpertemperatur, in dem Frühwarnsystem zur Hypoxieerkennung mit berücksichtigen.

In manchen Ausführungsformen können solche Module wie Gasmessmodule, Messmodule, Module zur Umgebungs- oder Umweltanalytik mindestens einen Energiespeicher, etwa eine Primärbatterie oder eine wiederaufladbare Batterie, aufweisen. Als Typen von wiederaufladbaren Batterien sind beispielsweise Typen von Lithium-Ionen-Batterien, Nickel-Metallhydrid-Batterien oder Nickel-Cadmium-Batterien bekannt. Als Typen von Primärbatterien sind beispielsweise Typen von Alkali-Mangan-Batterien, Silberoxid-Zink-Batterien, Lithiumbatterien, Aluminium-Luft-Batterien bekannt.

In Ausführungsformen mit wiederaufladbaren Batterien können zumeist Batterie-Ladesysteme und/oder Batteriemanagementsysteme zur Überwachung von Batterieladung und/oder Batteriezustand wie auch Schnittstellen zur Versorgung der Batterie-Ladesysteme und/oder Batteriemanagementsysteme mit elektrischer Ladeenergie zusätzlich mit in das Überwachungssystem eingebunden sein.

Zumeist weisen Batteriemanagementsysteme Schnittstellen zur Kommunikation nach Extern auf, um beispielsweise Daten oder Informationen zum Zustand der Batterie bereitstellen zu können, solche Schnittstellen können als drahtgebundene (z.B. CAN-Bus), kontaktlose (z.B. RFID, NFC), drahtlose (z.B. Bluetooth) oder infrarot-optische (z.B. IrDA) ausgestaltet sein. Zusätzlich können solche Module wie Gasmessmodule, Messmodule, Module zur Umgebungs- oder Umweltanalytik weitere Komponenten aufweisen, wie beispielsweise Komponenten zur Signalerfassung (ADµC), Signalverstärkung, zu analoger und/oder digitaler Signalverarbeitung (ASIC), Komponenten zu analoger und/oder digitaler Signalfilterung (DSP, FPGA, GAL, µC, µP), Signalumwandlung (A/D-Wandler), Komponenten (µC, µP) zur Kontrolle, Steuerung, Regelung, Komponenten (µC, µP) zur Ablaufsteuerung des Betriebes und zur Anwender-Interaktion aufweisen, Ein- und Ausgabe-Schnittstellen, Anwender-Schnittstelle (User-Interface) mit zumindest einem Bedienungselement und/oder mindestens einem Anzeigeelement.

Das mindestens eine Bedienungselement wie auch das mindestens eine Anzeigeelement können in oder an dem Überwachungssystem angeordnet sein oder dem Überwachungssystem zugeordnet sein.

Mit Hilfe des mindestens einen Bedienungselementes kann dem Anwender in manchen Ausführungsformen beispielsweise eine Bedienung mit Beginn (Start, Aktivierung) oder Ende (Stopp, Deaktivierung) des Überwachungssystems, eine Auswahl zwischen verschiedenen Betriebsarten des Überwachungssystems, eine Durchführung von Wartungs-, Justierungs- oder Kalibrationsverfahren ermöglicht sein.

Mit Hilfe des mindestens einen Anzeigeelementes kann es dem Anwender in manchen Ausführungsformen ermöglicht sein, über Ereignisse, Situationen, aktuelle Messwerte und/oder zeitlich zurück liegende Messwerte informiert zu werden, welche mittels der Sensoren oder Messmodule, insbesondere der Sauerstoffsensoren und/oder der Kohlenstoffdioxidsensoren bzw. der Sauerstoffmessmodule und/oder Kohlenstoffdioxidmessmodule messtechnisch erfasst und bereitgestellt worden sind.

Mittels der Anzeigeelemente können dem Anwender zudem von den Messwerten abgeleiteten Messgrößen wie beispielsweise Maximal- oder Minimalwerte Mittelwerte, Trends, Statistiken, Ereignisse, Alarmsituationen bereitgestellt werden.

Zudem können mittels der Anzeigeelemente dem Anwender allgemeine Informationen zum aktuellen Betriebszustand des Überwachungssystems wie Batterieladezustand, Batterie-Restlaufzeit, Wartungsinformationen, Informationen zum Überwachungssystem selbst wie Typ, Bezeichnung, Variante, Version, Seriennummer, Erstinbetriebnahme, anstehende Wartungsintervalle, Statusdaten, Betriebszustand (Ready, In-OP, Stand-by), Informationen zu Fehlfunktionen, Fehlerspeicher wie auch Bedienungshinweise gegeben werden.

Die Anzeigeelemente können in manchen Ausführungsformen als grafische Benutzerschnittstelle (Graphical User Interface, GUI) ausgebildet sein.

Zusätzlich zu den Anzeigeelementen können in manchen Ausführungsformen Eingabeelemente vorgesehen sein. Die Eingabeelemente können als mechanische oder berührungssensitive Taster oder Schalter, Dreh- oder Schieberegler wie auch in Form einer grafischen Benutzerschnittstelle (Graphical User Interface, GUI) ausgebildet sein.

In manchen Ausführungsformen können Anzeigeelemente mit Eingabeelementen kombiniert ausgeführt sein. In einer Ausgestaltung mit einem berührungssensitiven Display (touch screen) ergeben sich beispielsweise Möglichkeiten der Gestaltung von wandlungsfähigen Anzeigemöglichkeiten und Bedienungsmöglichkeiten, etwa einer Verwendung von Gesten (Wischen, Ziehen), um damit die Art der Anzeige zu variieren, beispielsweise um Darstellungselemente zu vergrößern oder zu verkleinern (Zoom-Funktion). Die Kombination von Anzeigeelementen mit Eingabeelementen kann vorzugsweise als grafische Benutzerschnittstelle (Graphical User Interface, GUI, Touch-Pad) ausgebildet sein.

In einigen Ausführungsformen ist ein Eingabeelement vorgesehen, das dem Anwender oder Benutzer des Überwachungssystems ermöglicht, bestimmte Situationen, definierte Aktionen oder Zustände an dem Überwachungssystem zu initiieren, zu annotieren, zu triggern, zu starten oder zu beenden. Ein solches Eingabeelement kann beispielsweise als ein Annotierungs-Taster und/oder als eine Paniktaste ausgestaltet sein, welches vorzugsweise durch Handbetätigung bedienbar ist. Alternativ kann auch eine Bedienung mit Sprachbefehl möglich sein, wobei dann der Annotierungs-Taster und/oder die Paniktaste entsprechend mit Mitteln zur Spracherfassung, Sprachverarbeitung und Spracherkennung mit Befehlserkennung ausgestaltet ist.

Das Eingabeelement kann erfindungsgemäß durch einen Beschleunigungssensor ausgebildet sein. Dazu können beispielsweise Daten oder Messwerte eines Beschleunigungssensors von der Kontrolleinheit mit verwendet werden, welcher als Bestandteil weiterer Sensorik an/in der Sensorik in dem Überwachungssystem zu einer Erfassung von Daten zur Ermittlung von situativen Parametern vorgesehen ist, um eine aktuelle Flugsituation mit Flughöhe, Flugrichtung, Fluggeschwindigkeit, Flugbeschleunigung, Fluglage mit Ausrichtung im Raum und Flugsituation bzw. Flugmanöver (z.B. Steigen, Sinken, Kurvenflug, Landeanflug, Start) im Überwachungssystem messtechnisch zu erfassen. Alternativ kann in oder an dem Überwachungssystem ein zusätzlicher 2- oder 3-Achsen-Beschleunigungssensor angeordnet sein, welcher in Verbindung mit der Kontrolleinheit eine Funktionalität eines Eingabeelementes darstellt.

Mit Hilfe des Beschleunigungssensors können erfindungsgemäß Bewegungen oder Auslenkungen des Überwachungssystems, Bewegungen oder Auslenkungen eines Gehäuses des Überwachungssystems, sowie auf den Beschleunigungssensor wirkende mechanische, taktile Reize oder taktile Anregungen sensorisch erfasst und als Messwerte oder Daten an die Kontrolleinheit bereitgestellt werden.

Erfindungsgemäß können mechanische oder taktile Reize oder Anregungen vom Flugzeugführer, Piloten oder Copiloten als Krafteinwirkung, Energie- oder Kraftzuführung in Richtung zumindest einer der sensorisch durch den Beschleunigungssensor erfassbaren Richtungen oder Achsen in Form von Handbewegungen auf das Überwachungssystem wirkender Betätigung einer Druckausübung, Schlageinwirkung (Push, Hit, Tapping) dem Beschleunigungssensor zugeführt.

Dies ist besonders dann vorteilhaft für den Flugzeugführer, Piloten oder Copiloten ermöglicht, wenn das Überwachungssystem als ein mobiles Modul in einer geschlossenen Tasche in oder an der Kleidung, beispielsweise an oder in einer Weste, Jacke oder einem Anzug, angeordnet ist.

Die Kraftzuführung kann somit erfindungsgemäß als eine Betätigungsaktivität durch die Kleidung hindurch auf den Beschleunigungssensor in dem Überwachungssystem erfolgen.

Die Verwendung des Beschleunigungssensors als Eingabemittel oder Eingabeelement ermöglicht erfindungsgemäß eine Betätigung oder Bedienung des Überwachungssystems durch den Flugzeugführer, Piloten oder Copiloten im Einsatz, wenn er anderweitige Eingabeelemente an dem als mobiles Modul ausgebildeten Überwachungssystem nicht erreichen kann.

Das ist beispielsweise dann der Fall, wenn das Überwachungssystem als ein mobiles Modul in einer Tasche in oder an der Kleidung angeordnet ist. Somit bietet der Beschleunigungssensor eine Alternative zu einer Bedienung des Überwachungssystems mittels einer manuellen Taste oder eines manuellen Schaltelementes. Für die Auswertung der Messwerte oder Daten des Beschleunigungssensors kann eine Kontrolleinheit verwendet werden, es kann jedoch auch eine zusätzliche Einheit vorgesehen sein, welche in dem Überwachungssystem dazu ausgerüstet ist, Daten des Beschleunigungssensors zu erfassen und auszuwerten.

Eine solche Auswertung der Daten des Beschleunigungssensors basiert im Wesentlichen auf Zeitmessungen. Mithilfe von Zeitmessungen in Verbindung mit Schwellenwerten für die Messwerte oder Daten des Beschleunigungssensors kann eine Auswertung hinsichtlich der Zeitdauer der taktilen Anregung wie auch hinsichtlich von Zeitdauern zwischen zwei oder mehr taktilen Anregungen des Beschleunigungssensors vorgenommen werden.

Die Kontrolleinheit kann erfindungsgemäß ausgebildet sein, anhand der Messwerte oder Daten des Beschleunigungssensors eine erstmalige Krafteinwirkung oder Kraftzuführung auf den Beschleunigungssensors mittels eines Vergleichs mit einem Schwellenwert zu erkennen. Wird ein Messwert des Beschleunigungssensors im Hinblick auf einen vorbestimmten Schwellenwert für eine erste vorbestimmte Zeitdauer überschritten, so interpretiert die Kontrolleinheit diese Situation als eine erste Krafteinwirkung in Richtung zumindest einer der sensorisch durch den Beschleunigungssensor erfassbaren Richtungen oder Achsen.

Aus diese Weise ist die Kontrolleinheit ausgebildet, anhand von Überschreitungen von Schwellenwerten für vorbestimmte Zeitdauern solche Krafteinwirkungen oder eine Folgen von Krafteinwirkungen als Eingabeaktivitäten des Piloten oder Copiloten zu erkennen. Damit ist ein Indiz für einen Beginn einer Eingabeaktivität des Flugzeugführers mittels Handbewegung als Eingabevorgang gegeben.

Werden anschließend die Messwerte des Beschleunigungssensors für einen zweiten vorbestimmten Zeitraum für einen zweiten vorbestimmten Schwellenwert abermals überschritten, so interpretiert die Kontrolleinheit diese Situation als Ende der ersten Krafteinwirkung. Erfolgt sodann innerhalb einer dritten vorbestimmten Zeitdauer eine Überschreitung eines Messwert des Beschleunigungssensors im Hinblick auf den ersten vorbestimmten Schwellenwert für die erste vorbestimmte Zeitdauer, so interpretiert die Kontrolleinheit diese Situation als weitere Krafteinwirkung in Richtung zumindest einer der sensorisch durch den Beschleunigungssensor erfassbaren Richtungen oder Achsen.

Damit ist ein Indiz für eine Fortsetzung der Eingabeaktivität des Flugzeugführers gegeben. Ergeben sich anschließend Unterschreitungen des zweiten vorbestimmten Schwellenwertes für einen zweiten vorbestimmten Zeitraum durch die Messwerte oder Daten des Beschleunigungssensors, so interpretiert die Kontrolleinheit diese Situation als Ende der weiteren Krafteinwirkung. Folgen innerhalb einer vierten vorbestimmten Zeitdauer keine weiteren Erfassungen und Erkennungen weiterer Krafteinwirkungen auf den Beschleunigungssensor, so ist ein Indiz für ein Ende der Eingabeaktivität des Flugzeugführers gegeben.

Mit dieser Art der Auswertung ist die Kontrolleinheit ausgebildet und in der Lage, mittels Auswertung der Messwerte des Beschleunigungssensors hinsichtlich des ersten, zweiten Schwellenwertes und der ersten, zweiten, dritten, vierten Zeitdauer im Betrieb des Überwachungssystems eine Eingabeaktivität eines "Zweifachen Drückens" (double tap) durch eine Handbewegung des Flugzeugführers zu erfassen und zu erkennen und kann darauf basierend in oder an dem Überwachungssystem dann weitere Aktionen auslösen. Solche Aktionen können in der Wirkungsweise beispielsweise Funktionen eines Annotierungs-Tasters und/oder einer Paniktaste entsprechen.

In manchen Ausführungsformen der Erfindung kann die Ausgestaltung der Kontrolleinheit zusätzlich zum "Zweifachen Drücken" (double tap) in Bezug auf die Auswertung derart erweitert werden, um auch Eingaben durch ein "Dreifaches Drücken" (triple tap) oder "Vierfachen Drücken" (quadruple tap) zu ermöglichen. Auf diese Weise ergibt sich eine einfache Kodierung für mittels des Beschleunigungssensors getätigte Eingaben, so dass zwischen verschiedenen Eingabesituationen mittels einer Fallunterscheidung zwischen "Zweifachem Drücken" (double tap), "Dreifachem Drücken" (triple tap) oder "Vierfachen Drücken" (quadruple tap) durch die Kontrolleinheit unterschieden werden kann.

Im Prinzip kann auch eine Eingabe eines "Einfachen Drückens" (single tap) ausgestaltet werden, dabei wäre die Zeitdauer der Anregung, welche das "Einfache Drücken" (single tap) indiziert, in einer Weise festzulegen, dass keine Verwechslungsmöglichkeiten mit anderweitigen Anregungen durch den Flugzeugführer, das Fluggerät oder Ausrüstung möglich sind.

In manchen Ausführungsformen der Erfindung kann die Ausgestaltung der Kontrolleinheit zusätzlich zur Auswertung von Zeitdauern der taktilen Anregungen auch Unterschiede von Zeitdauern zwischen den taktilen Anregungen des Beschleunigungssensors in die Auswertung mit einbeziehen. Diese Unterschiede können von der Kontrolleinheit dann zusätzlich zu Auswertungen von Formen des "mehrfachen Drückens" dazu genutzt werden, die Anzahl von voneinander unterscheidbarer Ereignisse zu erhöhen, beispielsweise durch eine Unterscheidung in eine "kurze Pause" und eine "lange Pause" als Zeitdauern zwischen den taktilen Anregungen des Beschleunigungssensors.

Somit ergibt sich durch diese Variation von Pausenlängen eine Art Morse- Code bei der Auswertung der taktilen Anregungen, welcher eine weitere Möglichkeit zu einer Kodierung von Ereignissen mit dem Beschleunigungssensor als Eingabeelement.

Es ergeben sich also Möglichkeiten und Vorteile dadurch, dass der Flugzeugführer, Pilot oder Copilot während des Flugbetriebes mit dem Beschleunigungssensor als Eingabeelement bei einer Unterscheidung von verschiedenen Eingabesituationen mit Zuordnung zu mit den Eingaben ausgelösten Handlungen oder Aktionen beispielsweise ein Messmanöver manuell starten kann, eine Eintragung (Annotierung) oder Zeitmarkierung in einem Logbuch setzen kann oder eine besondere gesundheitliche Situation, etwa ein Schwindelgefühl bei der Datenaufzeichnung oder Datenspeicherung, markieren kann, ohne einen Taster oder Schalter an dem Überwachungssystem mit Sichtkontakt zum Überwachungssystem bedienen zu müssen.

Ein Messmanöver kann beispielsweise durch das Messmanöver zur Bestimmung statischer und dynamischer Druckniveaus im pneumatischen System oder ein Messmanöver zur Aktivierung des Umschaltventils zur Erfassung von Gaskonzentrationen in der Kabine gegeben sein.

In manchen Ausführungsformen kann ein Datenspeicher zur Speicherung von Messwerten von, von den Messwerten abgeleiteten Messgrößen wie beispielsweise Maximal- oder Minimalwerte, Mittelwerte, Trends, Statistiken, Ereignisse, Alarmsituationen in oder an dem Überwachungssystem angeordnet oder dem Überwachungssystem zugeordnet sein. Ein solcher Datenspeicher kann als ein flüchtiger oder ein nichtflüchtiger Speicher (RAM, ROM, EEPROM) ausgebildet sein und sowohl als ein fester Bestandteil des Überwachungssystems oder auch als ein entfernbares und/oder transportables Speichermodul (USB-Stick, SD-Card) ausgebildet sein. Der Datenspeicher kann der Datenaufzeichnung oder Datenspeicherung von mittels des Eingabeelements vorgenommenen Eingaben dienen und dazu Funktionen eines Logbuches, Flugschreibers bereitstellen. Das Logbuch kann dabei in vorteilhafter Weise in einer Ausgestaltung mit Tabellen, Listen, Datensätzen eine Auswertung von Gaskonzentrations-Messwerten, Flow-Messwerten, Druck-Messwerten, Temperatur-Messwerten im Zeitverlauf mit zeitlicher Zuordnung und Markierung (Annotierung) weitere Ereignisse oder manuelle Eingaben mittels des Eingabeelementes aufnehmen und für eine gelichzeitige oder nachfolgende Auswertung bereithalten oder bereitstellen. Bei der Eintragung der Ereignisse können Messwerte oder Messsignale des Beschleunigungssensors hinzugezogen werden, um die jeweilige zu dem Zeitpunkt der durch den Piloten vorgenommenen Markierung/Notierung im Logbuch in einen Kontext zu Flugsituationen bzw. zu Flugmanövern für eine Auswertung in Echtzeit oder im Nachhinein zu setzen.

Bei der Eintragung der Ereignisse können Messwerte oder Messsignale eines Höhensensors hinzugezogen werden, um die jeweilige zu dem Zeitpunkt der durch den Piloten vorgenommenen Markierung/Notierung im Logbuch in einen Kontext zu Flugsituationen (Flughöhe) für eine Auswertung in Echtzeit oder im Nachhinein zu setzen. Bei Eintragung der Ereignisse können Messwerte oder Daten der Gassensorik hinzugezogen werden, um die jeweilige zu dem Zeitpunkt der durch den Piloten vorgenommenen Markierung/Notierung im Logbuch in einen Kontext zur Gasversorgung (CO₂, O₂) für eine Auswertung in Echtzeit oder im Nachhinein zu setzen.

In einer besonderen Ausgestaltung mancher Ausführungsformen kann die Kontrolleinheit ausgebildet sein, bei einer Durchführung der Signalverarbeitung und/oder Signalfilterung der Messwerte der Sensorik einen Umgebungsparameter und/oder einen situativen Parameter mit zu berücksichtigen und/oder mit in eine Anpassung der Signalverarbeitung einzubeziehen.

In manchen Ausführungsformen kann die Kontrolleinheit ausgebildet sein, bei der Organisation der Alarmierung vorbestimmte Schwellenwerte zu verwenden, welche für bestimmte Werte von Gaskonzentrationen, insbesondere Konzentrationen an Sauerstoff oder Kohlenstoffdioxid, Kohlenstoffmonoxid, im Datenspeicher des Überwachungssystems hinterlegt sein können.

In manchen Ausführungsformen kann die Kontrolleinheit ausgebildet sein, ein Frühwarnsystem zur Hypoxie-Erkennung auf Basis von aktuellen und zeitlich zurückliegenden Messwerten der Sensorik, beispielsweise in Form einer Trendüberwachung von Konzentrationen an Sauerstoff und/oder Kohlenstoffdioxid
- mit Hilfe einer Entscheidungsmatrix
- oder mit Hilfe speziell angepasster Algorithmen
- oder mit Hilfe von lernfähigen oder selbstlernenden Algorithmen (z.B.: SVM, Random Forest, AI, Deep-Learning, ICA, PCA)
zur Anwendung zu bringen.

In besonderen Ausgestaltungen mancher Ausführungsformen kann die Kontrolleinheit dabei einen Umgebungsparameter und/oder einen situativen Parameter mit berücksichtigen.

In weiteren besonderen Ausgestaltungen mancher Ausführungsformen kann die Kontrolleinheit ein an das Frühwarnsystem angepasstes Alarmmanagement anwenden.

In einer besonderen Ausgestaltung mancher Ausführungsformen kann die Kontrolleinheit ausgebildet sein, weitere beispielsweise mittels der Datenschnittstelle oder von dem Überwachungssystem zugeordneten Messsystemen bereitgestellte, physiologische Daten von Luftfahrzeugführern, Flugzeugführern, Piloten, Copiloten beispielsweise etwa EKG, Herzrate, Variabilität der Herzrate, Sauerstoffsättigung im Blut, Körpertemperatur in dem Frühwarnsystem zur Hypoxie-Erkennung mit zu berücksichtigen.

In manchen Ausführungsformen erfolgt durch die Kontrolleinheit in einem ersten Schritt eine Aktivierung der Sensorik des Überwachungssystems, in einem zweiten Schritt erfolgt eine Vorbereitung einer Datenspeicherung mit Initialisierung eines Datenspeichers.

In einem dritten Schritt erfolgt mit einer zeitlichen Steuerung eine messtechnische Erfassung von Messwerten der Sensorik des Überwachungssystems.

In einem vierten Schritt erfolgt eine Datenspeicherung der Messwerte in dem Datenspeicher mit einer zugehörigen Zeitinformation.

Der dritte und der vierte Schritt werden von der Kontrolleinheit bis zu einer Beendigung des Verfahrens zum Betrieb eines Überwachungssystems kontinuierlich fortgesetzt.

In manchen Ausführungsformen kann bei der Datenspeicherung der Messwerte der Sensorik mit der zugehörigen Zeitinformation in dem Datenspeicher eine zusätzliche Speicherung von situativen Parametern und/oder Umgebungsparametern ermöglicht sein.

In manchen Ausführungsformen kann bei einer Aktivierung eines Eingabeelementes durch einen Anwender eine - von der zeitlichen Steuerung unabhängige - zusätzliche Erfassung von Messwerten der Sensorik zum Zeitpunkt der Aktivierung des Eingabeelementes ermöglicht sein.

Weitere, die Erfindung verbessernde Maßnahmen ergeben sich aus der nachfolgenden Beschreibung zu einigen Ausführungsbeispielen der Erfindung, welche in den Figuren dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen, können sowohl für sich als auch in den verschiedenen Kombinationen erfindungswesentlich sein.

Es zeigen jeweils schematisch:
- die Figuren 1a, 1b ein Überwachungssystem mit einer Sensorik,
- die Figuren 2a und 2b ein Überwachungssystem nach den Figuren 1a, 1b mit einer Messfunktionalität für Sauerstoff und Kohlenstoffdioxid,
- die Figur 3 eine Erweiterung der Varianten nach den Überwachungssystemen gemäß den Figuren 1a, 1b, 2a, 2b,
- die Figuren 4 und 5 zwei Varianten der Überwachungssysteme nach den Figuren 1a, 1b, 2a, 2b, 3 mit weiterer Sensorik,
- die Figur 6 eine Variante des Überwachungssystems nach der Figur 3,
- die Figur 7 eine weitere Variante des Überwachungssystems nach der Figur 3,
- die Figur 8 eine alternative Variante des Überwachungssystems nach der Figur 6.

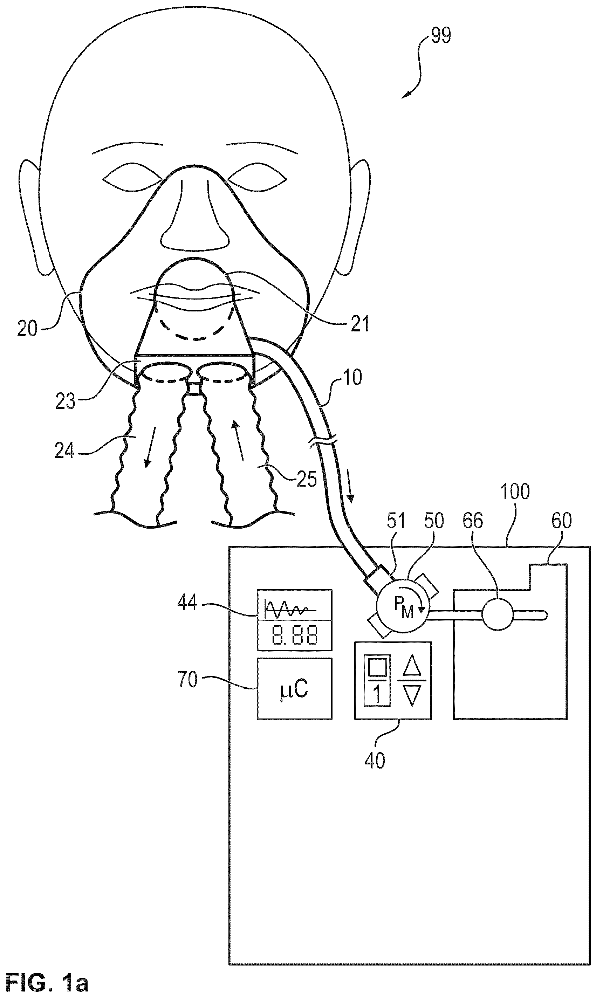

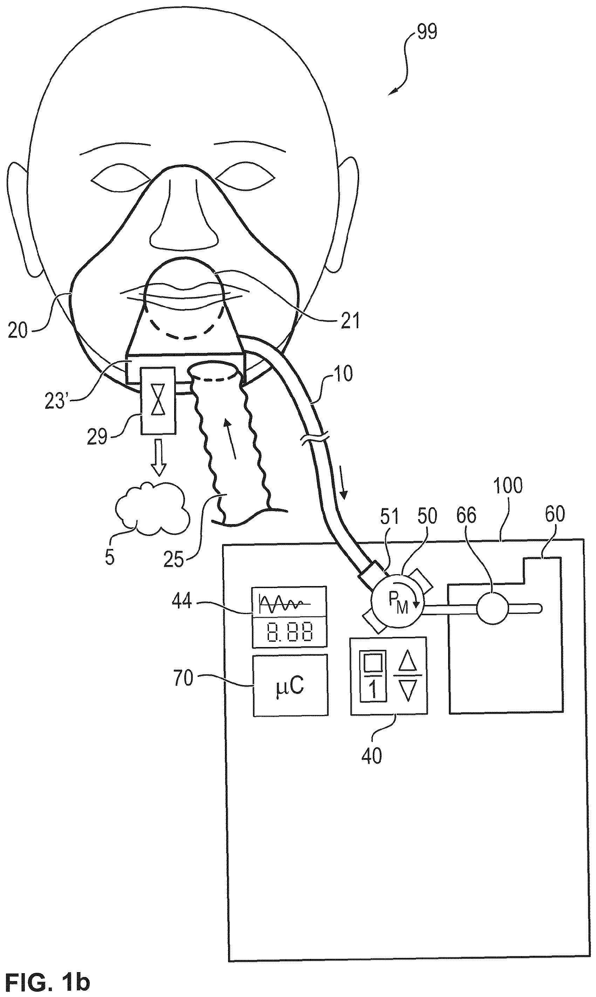

Die Figuren 1a, 1b zeigen ein Überwachungssystem 100, welches mit einer Messgasleitung 10 mit einer Atemmaske 20 einer Person 99 verbunden ist.

Gleiche Elemente in den Figuren 1a, 1b sind in den Figuren 1a, 1b mit den gleichen Bezugszeichen bezeichnet.

Die Person 99 stellt in dieser Figur 1 einen Flugzeugführer (Piloten, Copiloten) eines Flugzeugs, insbesondere eines Strahlflugzeugs (Jet) dar. Die Atemmaske 20 weist einen Gasanschluss 21, ein Anschlusselement 23 sowie Schlauchleitungen 24, 25 auf. Die Schlauchleitungen 24, 25 dienen der Abfuhr und Zufuhr von Atemgasen zu der Person 99.

In dieser Figur 1a sind die Schlauchleitungen als zwei separate Schlauchleitungen 24, 25 gezeigt. Wie in der Figur 1b gezeigt, sind aber auch Ausführungen mit einem Anschlusselement 23' möglich, in denen nur eine Schlauchleitung 25 zu einer Gaszufuhr von Atemgas zur Einatmung vorhanden ist und die Ausatmung über ein Ausatemventil 29 in der Atemmaske 20 an eine Umgebung 5 erfolgt. Eine weitere Möglichkeit ist mit einer Realisierung eines koaxialen Schlauchsystems gegeben, welche zwei Schlauchleitungen 24, 25 als gemeinsames Element aufweist.

Die Fortführung bzw. Zufuhr von Atemgasen und die dazu erforderlichen Mittel oder Elemente in das Luftfahrzeug oder Fluggerät zur Bereitstellung des Atemgases sind aus Gründen einer übersichtlichen Darstellung in dieser Figur 1a und den übrigen Figuren nicht mit gezeigt. Das Überwachungssystem 100 weist Bedienungselemente 40, Anzeigeelemente 44, mindestens ein Modul zur Gasförderung 50, eine Sensorik 60 mit mindestens einem Sensor 66 auf. Das Modul zur Gasförderung 50 ist vorzugsweise als eine Pumpe P_{M}, weiter vorzugsweise als eine die piezo-elektrisch betriebene Pumpe P_{M} ausgebildet.

Zudem weist das Überwachungssystem 100 eine Kontrolleinheit 70 auf. Bedienungselemente 40, die Anzeigeelemente 44, die Sensorik 60, das Modul zur Gasförderung 50 sind mit der Kontrolleinheit 70 über Signal- und Datenleitungen oder Kontrollleitungen verbunden. Diese Kontrollleitungen bzw. Signal- und Datenleitungen können beispielsweise als ein Bussystem (CAN) oder Netzwerk ausgestaltet sein.

Diese Kontrollleitungen bzw. Signal- und Datenleitungen werden aus Gründen einer übersichtlichen Darstellung in der Figur 1a sowie den weiteren Figuren nicht gezeigt.

Die Kontrolleinheit 70 ist ausgebildet und dazu vorgesehen, das Modul zur Gasförderung 50 derart zu kontrollieren und/oder anzusteuern, dass eine Förderung von Atemgasen von der Atemmaske 20 durch die Messgasleitung 10 und einen Gaseinlass 51 zu der Sensorik 60 zustande kommt. Somit steht dann dem mindestens einen Sensor 66 in der Gassensorik 60 eine Menge oder Teilmenge an Atemgas zur Verfügung, um diese messtechnisch zu erfassen und/oder zu analysieren und der Kontrolleinheit 70 als Messwerte bereitzustellen. Mittels der Kontrolleinheit 70 wird ermöglicht, die Messwerte auszuwerten, aufzubereiten und zumindest auf Teileelementen der Anzeigeelemente 44 zur Darstellung zu bringen.

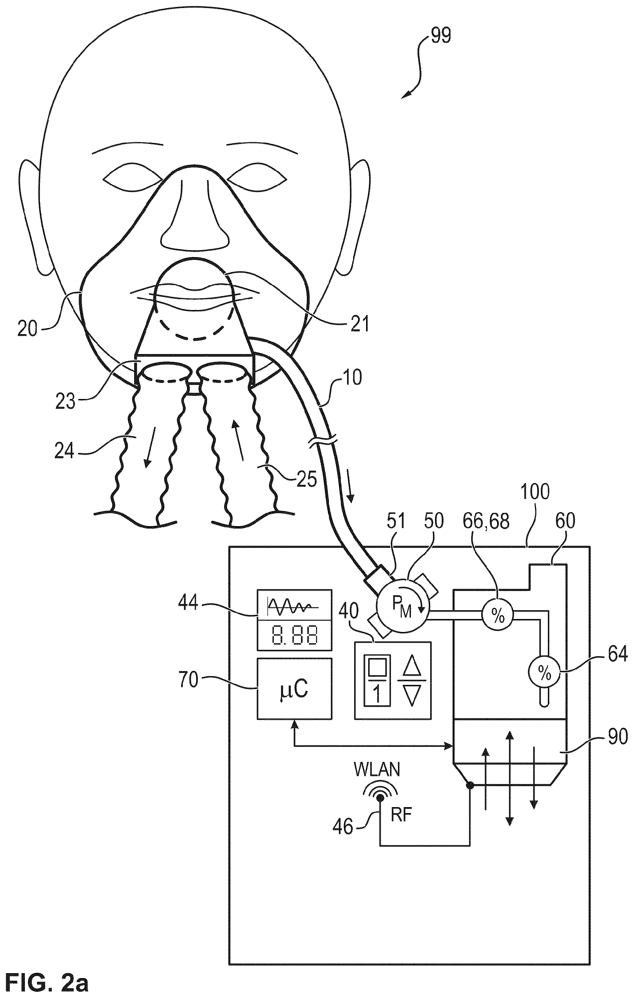

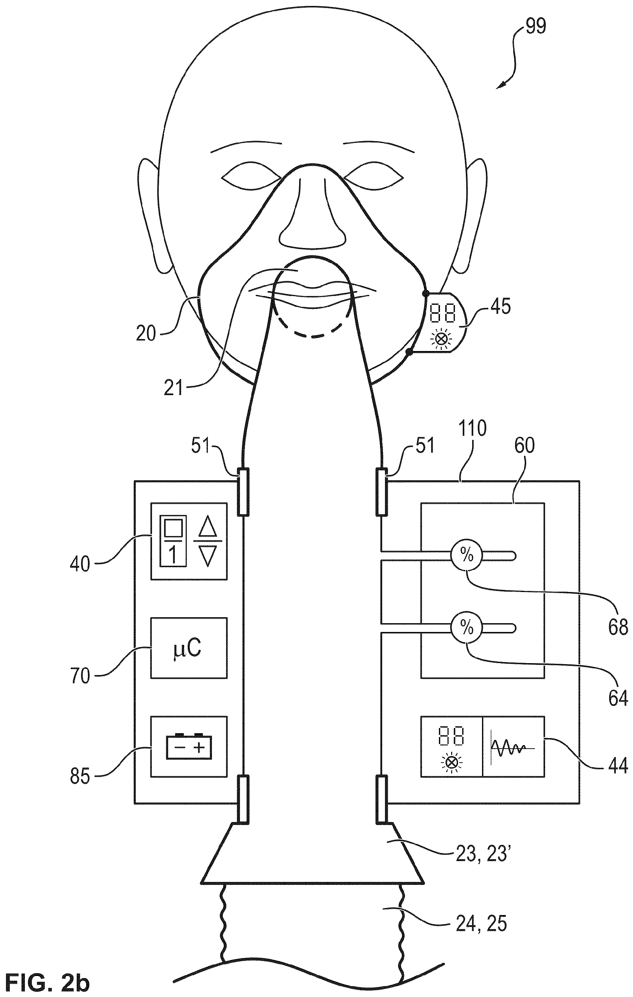

Die Figuren 2a, 2b zeigen Überwachungssysteme 100, 110 gemäß den Figuren 1a, 1b mit der Besonderheit, dass der Sensor 66 in der Sensorik 60 als ein Sauerstoffsensor 68 ausgebildet ist und zudem ein weiterer Sensor als ein Kohlenstoffdioxidsensor 64 ebenfalls mit in der Sensorik 60 angeordnet ist. Gleiche Elemente in den Figuren 1a, 1b, 2a, 2b sind in den Figuren 1a, 1b, 2a, 2b mit den gleichen Bezugszeichen bezeichnet.

Die Figur 2b zeigt eine Variante 110 eines Überwachungssystems nach der Figur 2a mit einem Sauerstoffsensor 68 und einem Kohlenstoffdioxidsensor 64, wobei das Überwachungssystem 110 ohne eine Messgasleitung 10 direkt an der Atemmaske 20 angeordnet oder als ein Teil der Atemmaske 20 ausgestaltet ist. Eine Pumpe P_{M} wie in den Varianten nach den Figuren 1a, 1b, 2a zur Förderung von Mengen an Atemgas von der Atemmaske 20 hin zu der Sensorik 60 kann dabei gegebenenfalls entfallen. Für den Fall, dass optional auch Mengen an Gas aus der Kabine oder dem Cockpit zu der Sensorik gefördert werden sollen, ist auch in der Anordnung nach der Figur 2b eine optionale Pumpe 56 in oder an der Sensorik angeordnet.

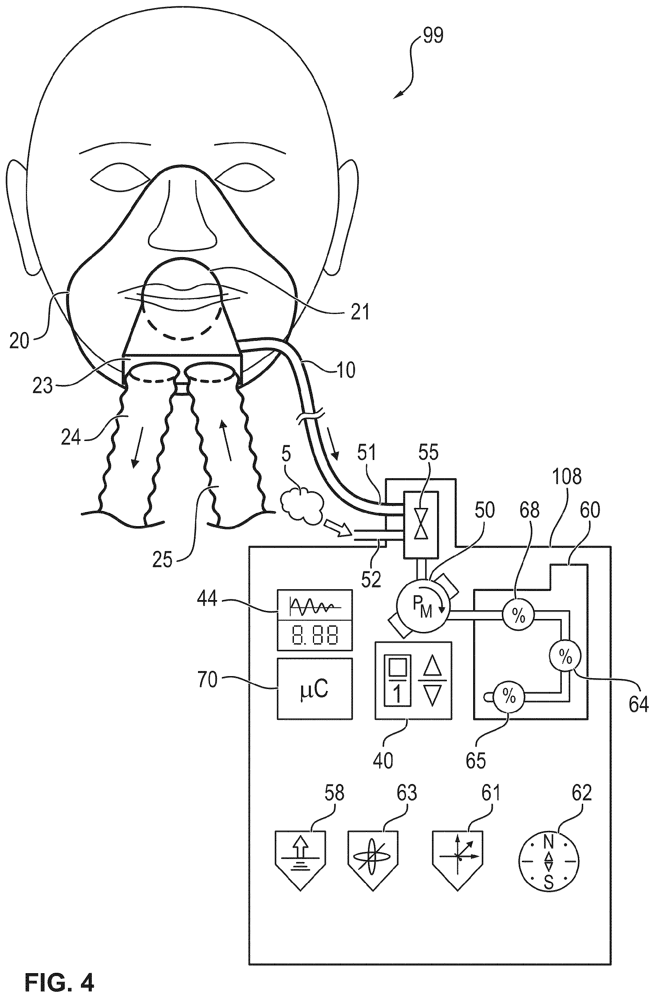

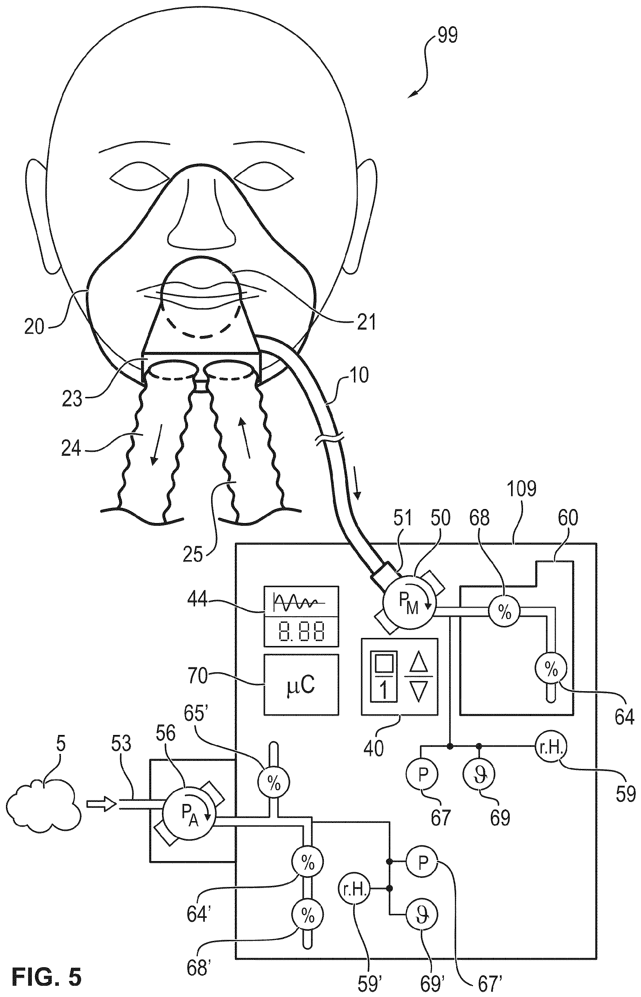

Die Anordnung einer solch optionalen Pumpe 56 in dem Überwachungssystem 110 ist aus Gründen einer übersichtlichen Darstellung nicht mit gezeigt. In der Figur 2b ist exemplarisch - in ähnlicher Ausgestaltung auch als optionale Komponente der Ausgestaltungen gemäß den Figuren 1a, 2a, 3, 4, 5 zu verstehen - ein Energiespeicher 85 gezeigt. Ein solcher Energiespeicher 85, ausgebildet als Primärbatterie oder aufladbare oder wiederaufladbare Batterie (rechargeable battery, Akkumulator), ist geeignet ausgebildet, die diversen Komponenten (60, 70, 40, 44, 75) der Überwachungssysteme 110, 108 (Figur 4), 109 (Figur 5), 100 (Figur 1a, Figur 1b, Figur 2a, Figur 3) mit elektrischer Energie zu versorgen.

In der Figur 1b ist - in ähnlicher Ausgestaltung auch als optionale Komponente der Ausgestaltungen gemäß den Figuren 1a, 2a, 3, 4, 5 zu verstehen - eine optionale Ausgestaltung mit einem an oder in der Maske 20 angeordneten weiteren Anzeigeelement 45 gezeigt. Dies weitere Anzeigeelement 45 ist mittels - aus Gründen der Übersichtlichkeit nicht gezeigten - Signal- oder Datenleitungen mit der Kontrolleinheit 70 verbunden. Diese weitere Anzeigeelement 45 kann zusätzlich oder alternativ zu dem Anzeigeelement 44 eingesetzt werden.

Die Ausgestaltung des weiteren Anzeigeelementes kann beispielsweise in Form eines In-Mask-Displays oder Head-Up-Displays realisiert sein. Die Figur 2a zeigt zusätzlich eine Datenschnittstelle 90, welche einerseits dazu ausgebildet sein kann, Daten von extern zu empfangen und diese Daten dann der Kontrolleinheit 70 bereitzustellen. Die der Datenschnittstelle zugehörigen Datenleitungen werden aus Gründen einer übersichtlichen Darstellung in der Figur 2a sowie den weiteren Figuren nicht gezeigt. Andererseits können mittels der Datenschnittstelle 90 beispielsweise Messwerte des Überwachungssystems 100 oder der Sensorik 60 nach extern bereitgestellt werden.

So können über diese Datenschnittstelle 90 - beispielsweise von Komponenten des Luftfahrzeuges oder Fluggerätes - aktuelle Umgebungsparameter oder situative Parameter zur Situation des Luftfahrzeuges oder Fluggerätes empfangen und der Kontrolleinheit 70 zur Berücksichtigung bei der Verarbeitung von Messwerten und/oder bei der Kontrolle der Pumpe P_{M} 50 bereitgestellt werden. Weiterhin können Messwerte und/oder von den Messwerten abgeleiteten Messgrößen oder Parameter wie auch Informationen oder Zustandsdaten von der Kontrolleinheit 70 mittels der Datenschnittstelle für Komponenten des Luftfahrzeuges oder Fluggerätes bereitgestellt werden. Auf diese Weise ist es beispielsweise möglich, Messwerte und/oder von den Messwerten abgeleiteten Messgrößen oder Parameter wie auch Informationen oder Zustandsdaten auf externen Anzeigeelementen des Luftfahrzeuges oder Fluggerätes zur Anzeige zu bringen.

Die Datenschnittstelle kann unidirektional oder bidirektional ausgebildet sein, beispielsweise drahtgebunden (CAN-Bus, LAN, Ethernet, RS485, NMEA183) oder drahtlos (WLAN, Bluetooth, NFC).

Als aktuelle Umgebungsparameter für eine Umgebungssituation des Luftfahrzeuges oder Fluggerätes sind beispielsweise zu nennen:
- Umgebungsdruck außerhalb von Cockpit oder Kabine
   des Luftfahrzeugs oder Fluggerätes
- Umgebungstemperatur innerhalb von Cockpit oder Kabine des Luftfahrzeugs oder Fluggerätes
- Gaszusammensetzung innerhalb von Cockpit oder Kabine
   des Luftfahrzeugs oder Fluggerätes
- absolute und/oder relative Feuchte innerhalbhalb von Cockpit oder Kabine des Luftfahrzeugs oder Fluggerätes
- Dichte und/oder Umgebungsdruck innerhalb von Cockpit oder Kabine des Luftfahrzeugs oder Fluggerätes
- Umgebungstemperatur innerhalb von Cockpit oder Kabine
   des Luftfahrzeugs oder Fluggerätes
- Gaszusammensetzung innerhalb von Cockpit oder Kabine
   des Luftfahrzeugs oder Fluggerätes
- Umgebungsdruck außerhalb von Cockpit oder Kabine
   des Luftfahrzeugs oder Fluggerätes
- Umgebungstemperatur außerhalb von Cockpit oder Kabine des Luftfahrzeugs oder Fluggerätes
- Gaszusammensetzung außerhalb von Cockpit oder Kabine des Luftfahrzeugs oder Fluggerätes
- absolute und/oder relative Feuchte außerhalb von Cockpit oder Kabine des Luftfahrzeugs oder Fluggerätes
- Dichte und/oder Umgebungsdruck außerhalb von Cockpit oder Kabine des Luftfahrzeugs oder Fluggerätes
- Umgebungstemperatur außerhalb von Cockpit oder Kabine des Luftfahrzeugs oder Fluggerätes
- Gaszusammensetzung außerhalb von Cockpit oder Kabine des Luftfahrzeugs oder Fluggerätes
- Druckniveau, Druckverlauf, Druck-Zeitverlauf,
   Druckunterschiede, Druckschwankungen in Atemgas, Atemgasgemisch oder in der Atemluft in der Zuführung zu Flugzeugführer, Pilot oder Copilot,
- Druckniveau, Druckverlauf, Druckunterschiede, Druckschwankungen
   in der Bereitstellung der Bordtechnik (z.B. von Gastanks, Drucksauerstoffflaschen, Luftansaugung, Gasaufbereitung, Filterung, Gasförderung) für Atemgas, Atemgasgemisch oder Atemluft.

Als situative bzw. aktuelle situative Parameter zur Situation des Luftfahrzeuges oder Fluggerätes sind beispielsweise zu nennen:
- eine Flugrichtung
- eine Flughöhe
- eine Flugachsenlage
- eine Fluglage,
   beispielsweise etwa Rückenflug, Kurvenflug, Sturzflug, Sinkflug, Steigflug
- eine Fluggeschwindigkeit
- eine Flugrichtung
- eine horizontale Beschleunigung
- eine vertikale Beschleunigung
- ein Gierwinkel oder ein Rollwinkel
- ein Restvorrat an Sauerstoff oder Luft
- ein Restvorrat an Sauerstoffdruckgas oder Druckluft.

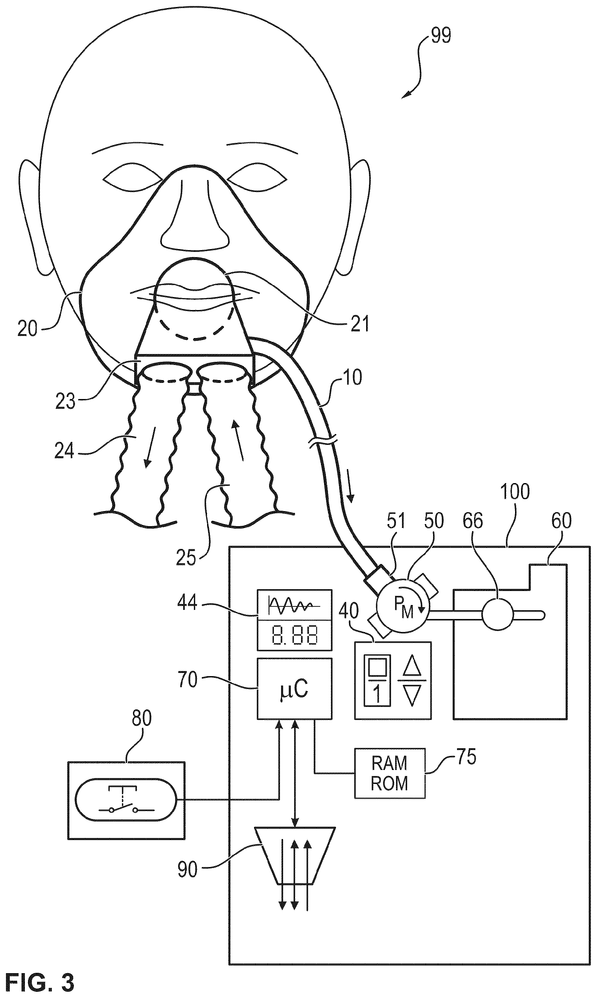

Die Figur 3 zeigt ein Überwachungssystem 100 gemäß Figur 1a, 1b, 2a mit der Besonderheit, dass ein Eingabeelement 80 mit einer Signal- oder Datenverbindung mit der Kontrolleinheit 70 an dem Überwachungssystem angeordnet ist. Gleiche Elemente in den Figuren 1a, 1b, 1c, 2, 3 sind in den Figuren 1a, 1b, 1c, 2, 3 mit den gleichen Bezugszeichen bezeichnet.

Über dies Eingabeelement 80 ist es dem Flugzeugführer, Piloten oder Copiloten ermöglicht, bestimmte Ereignisse oder Situationen des Flugbetriebes zu markieren wie auch bestimmte persönliche, beispielsweise gesundheitliche Ereignisse, Situationen oder Symptome, etwa Fieber, Herzrasen oder ein Schwindelgefühl, im Zeitverlauf des Einsatzes zu markieren. Diese Markierung kann von der Kontrolleinheit 70 dazu genutzt werden, die Ereignisse oder Situationen mit einer Zeitinformation zu kombinieren und die Kombination von Zeitinformation, Ereignis bzw. Situation dann in einem Datenspeicher 75 abzulegen. Der Datenspeicher 75 kann als flüchtiger oder nichtflüchtiger Speicher (RAM, ROM, EEPROM) ausgebildet sein und sowohl als ein fester Bestandteil oder als entfernbares Speichermodul (USB-Stick, SD-Card) in oder an dem Überwachungssystem 100, 110 (Figur 2b) angeordnet sein.

Auch kann eine Bereitstellung und/oder ein Austausch der Daten mit einer externen - in den Figuren nicht gezeigten - Auswerteeinheit ermöglicht sein, beispielsweise mittels einer Datenschnittstelle 90 in ähnlicher Ausgestaltung, wie in der Figur 2b gezeigt und beschrieben.

Dieses Eingabeelement 80 kann damit dazu dienen, die erfassten Messwerte der Sensorik 60 und die Ereignisse und Situationen des Flugbetriebes um weitere Informationen, die mittels des Eingabeelements von Flugzeugführer, Piloten oder Copiloten bereitgestellt werden, zu ergänzen und mit einer Zeitinformation, beispielsweise in Form eines Zeitstempels, zu versehen. Es ist aber auch eine Ausgestaltung des Eingabeelementes als Paniktaste möglich, welche dem Flugzeugführer, Piloten oder Copiloten unmittelbar ermöglicht, sich in einer aus seiner Wahrnehmung heraus besonderen Situation, beispielsweise einer Situation mit besonderer, objektiv oder subjektiv empfundener Gefahrenlage oder einer Risikosituation, bemerkbar zu machen. Die markierten Messwerte und/oder Ereignisse, Situationen und auch die besonderen Situationen können beispielsweise mittels der Datenschnittstelle 90 nach unmittelbar extern bereitgestellt werden und ggf. - ebenfalls unmittelbar (online) - über ein Kommunikationssystem des Luftfahrzeuges oder Fluggerätes an eine Bodenstation oder an andere Luftfahrzeuge oder Fluggeräte übermittelt werden. Weiterhin ist eine Auswertung der markierten Messwerte und/oder Ereignisse, Situationen und besonderen Situationen nach dem Einsatz im Nachhinein (offline) mittels des Datenspeichers 75 und/oder der Datenschnittstelle 90 ermöglicht.

Die Figuren 4 und 5 zeigen Varianten der Überwachungssysteme 100, 110 nach den Figuren 1a, 1b, 2a, 2b, 3 mit weiteren Komponenten der Sensorik 60. Die zugehörigen Kontrollleitungen bzw. Signal- und Datenleitungen für die weiteren Sensoren der Sensorik 60 werden aus Gründen einer übersichtlichen Darstellung in den Figuren 4 und 5 nicht gezeigt. Gleiche Elemente in den Figuren 1a, 1b, 1c, 2, 3, 4, 5 sind in den Figuren 1a, 1b, 1c, 2, 3, 4, 5 mit den gleichen Bezugszeichen bezeichnet.

Diese weiteren Sensoren in der Sensorik 60 können zu einer Bestimmung von aktuellen Umgebungsparametern innerhalb und/oder außerhalb von Cockpit oder Kabine des Luftfahrzeuges oder Fluggerätes und/oder zu einer Bestimmung von aktuellen situativen Parametern und Situationen wie auch zu einer Bestimmung von physikalischen Eigenschaften und zu einer weiteren Bestimmung der Zusammensetzung des Atemgases dienen.

Als weitere Komponenten der Sensorik 60 sind in dem Überwachungssystem 108 in der Figur 4 exemplarisch, welche auch als optionale Möglichkeiten der Ausgestaltung für die Figuren 1a, 1b, 2a, 2b, 3, 5 zu verstehen sein sollen, folgende weitere Sensoren gezeigt:
- mindestens ein Beschleunigungssensor 61 in Form eines 2- oder 3-Achsen-Beschleunigungssensors (Accelerometer)
- mindestens ein Kompass-Sensor 62,
   beispielsweise ein elektronischer Kompass,
   Kreiselkompass oder Fluxgate-Kompass
- mindestens ein Höhensensor 58
- mindestens ein Gyro-Sensor 63.

Als weitere Komponenten der Sensorik 60 sind in der Figur 5 exemplarisch,
welche auch als optionale Möglichkeiten der Ausgestaltung für die Figuren 1a, 1b, 2a, 2b, 3, 4 zu verstehen sein sollen,
folgende weitere Sensoren gezeigt:
   - mindestens ein Temperatursensor 69, 69'
   - mindestens ein Drucksensor 67, 67'
   - mindestens ein Feuchtesensor 59, 59'

Die weiteren Sensoren in der Sensorik 60 können als Drucksensoren ausgestaltet sein, welche dazu ausgebildet und dazu vorgesehen sein können, einen Umgebungsdruck aus der Umgebung, insbesondere einen Druck oder eine Dichte innerhalb und/oder außerhalb von Cockpit oder Kabine des Luftfahrzeugs oder Fluggerätes messtechnisch zu erfassen und der Kontrolleinheit 70 bereitzustellen.

Die weiteren Sensoren in der Sensorik 60 können als Temperatursensoren ausgestaltet sein, welche dazu ausgebildet und dazu vorgesehen sein können, eine Umgebungstemperatur der Umgebung, insbesondere eine Temperatur innerhalb und/oder außerhalb von Cockpit oder Kabine des Luftfahrzeugs oder Fluggerätes, messtechnisch zu erfassen und der Kontrolleinheit 70 bereitzustellen. Diese weiteren Sensoren in der Sensorik 60 können als Feuchtesensoren zu einer Erfassung einer absoluten oder relativen Feuchte der Umgebung ausgestaltet sein, welche dazu ausgebildet und dazu vorgesehen sein können, eine Feuchtigkeit in der Umgebung, insbesondere innerhalb und/oder außerhalb von Cockpit oder Kabine des Luftfahrzeugs oder Fluggerätes messtechnisch zu erfassen und der Kontrolleinheit 70 bereitzustellen.

Die weiteren Sensoren in der Sensorik 60 können als mindestens ein weiterer Gassensor 65 zu einer Erfassung einer Gaszusammensetzung der Umgebung ausgestaltet sein, welche dazu ausgebildet und dazu vorgesehen sein können, eine Gaszusammensetzung in der Umgebung, insbesondere innerhalb und/oder außerhalb von Cockpit oder Kabine des Luftfahrzeugs oder Fluggerätes, messtechnisch zu erfassen und der Kontrolleinheit 70 bereitzustellen. Als weitere Gassensoren können elektrochemische Gassensoren, katalytische Gassensoren, optische, infrarot-optische Gassensoren, Photo-Ionisations-Gassensoren, Festkörperelektrolyt-Gassensoren oder Halbleiter-Gassensoren zum Einsatz kommen, um das Atemgas zusätzlich zur messtechnischen Erfassung von Konzentrationen an Sauerstoff und Kohlenstoffdioxid auch noch hinsichtlich weiterer Substanzen, wie etwa Kohlenstoffmonoxid, Kohlenstoffwasserstoffen, Rückständen oder Produkten von Verbrennungsprozessen, überwachen zu können. Ein in der Figur 4 gezeigtes Umschaltventil 55, beispielsweise ausgebildet als Ventilmodul oder als ein Teil eines Ventilmoduls, ermöglicht eine Umschaltung von Mengen oder Teilmengen an Gasproben zwischen dem Gaseinlass 51 und einem weiteren Gasanschluss 52. Damit ist es ermöglicht, mittels der Pumpe P_{M} 50 einerseits Atemgas von der Atemmaske 20 zu der Sensorik 60 zu fördern, es ist aber zudem auch möglich, mittels der Pumpe P_{M} 50 Mengen an Gas oder Gasgemisch aus einer Umgebung 5 zu der Sensorik 60 zu fördern und mittels der Sensorik 60 messtechnisch zu erfassen. Das Umschaltventil 55 wird von der Kontrolleinheit 70 kontrolliert. So kann Außenluft von außerhalb des Luftfahrzeuges oder Fluggerätes oder Innenluft aus Kabine oder Cockpit des Luftfahrzeuges oder Fluggerätes über den weiteren Gasanschluss 52 zugeführt werden und abwechselnd - mit Kontrolle der Kontrolleinheit 70 - eine Überwachung von Gaskonzentrationen in der Atemmaske 20, Cockpit, Kabine oder Außenluft ermöglicht sein.

Die in der Figur 5 in dem Überwachungssystem 109 zusätzlich zu den weiteren Gassensoren 65 und Sensoren 59, 67, 69 dargestellten noch weiteren Sensoren 59', 64', 68', 69' und mindestens einem weiteren Gassensor 65' sind mit einer weiteren Pumpe P_{A} 56 pneumatisch oder fluidisch verbunden. Gleiche Elemente in den Figuren 1a, 1b, 1c, 2, 3, 4, 5 sind in den Figuren 1a, 1b, 1c, 2, 3, 4, 5 mit den gleichen Bezugszeichen bezeichnet. Diese weitere Pumpe P_{A} 56 ermöglicht eine Zuführung von Gas aus einer Umgebung 5 über einen weiteren Gasanschluss 53, beispielsweise von Außenluft von außerhalb des Luftfahrzeuges oder Fluggerätes oder von Innenluft aus Kabine oder Cockpit des Luftfahrzeuges oder Fluggerätes. Die weitere Pumpe P_{A} 56 wird von der Kontrolleinheit 70 kontrolliert. So kann Außenluft von außerhalb des Luftfahrzeuges oder Fluggerätes oder Innenluft aus Kabine oder Cockpit des Luftfahrzeuges oder Fluggerätes über den weiteren Gasanschluss 53 zugeführt werden.

So ist eine gleichzeitige Überwachung von Gaskonzentrationen in der Atemmaske 20 und von Gaskonzentrationen in Cockpit, Kabine oder Außenluft ermöglicht. Weitere Sensoren in der Sensorik 60 können ausgebildet sein, die aktuelle Situation des Luftfahrzeuges oder Fluggerätes messtechnisch zu erfassen. So kann mittels der Daten eines Beschleunigungssensors 61, vorzugsweise ausgebildet als 3-Achsen-Beschleunigungssensor (3-axis-accelerometer) in Kombination mit einem Höhen-Sensor 58 (Altimeter), Gyro-Sensor 63 und optionale Hinzuziehung von Informationen eines Kompass-Sensors 62 eine aktuelle Flugsituation mit Flughöhe, Flugrichtung, Fluggeschwindigkeit, Flugbeschleunigung, Fluglage mit Ausrichtung im Raum (XYZ-Orientation) und Flugsituation bzw. Flugmanöver (z.B. Steigen, Sinken, Kurvenflug, Landeanflug, Start) von der Kontrolleinheit (70) ermittelt werden.

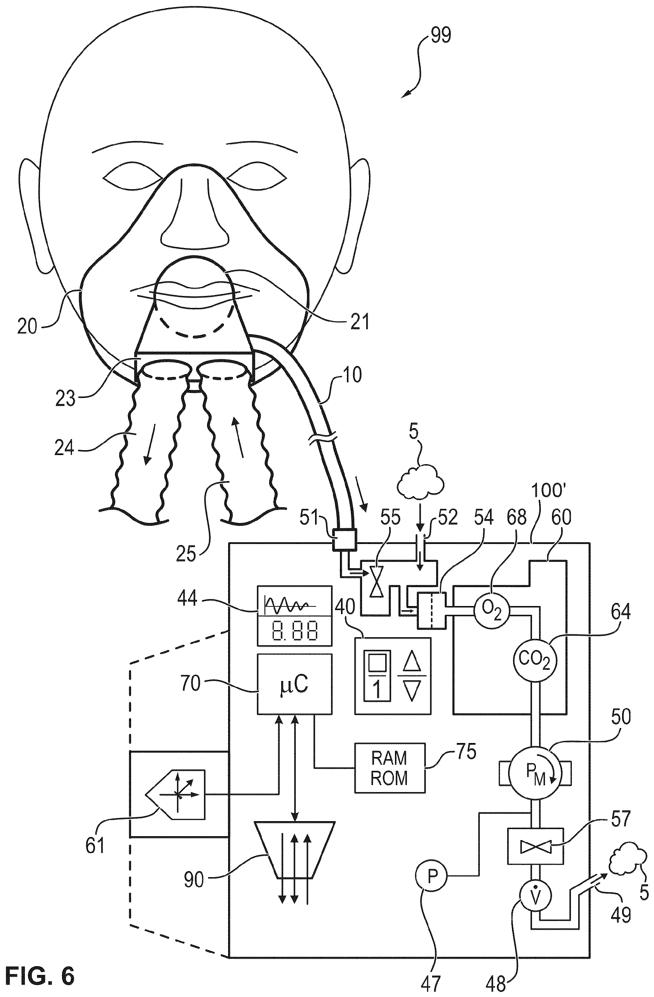

Die Figur 6 zeigt eine Variante in Abwandlung der Figur 3, wobei die Pumpe P_{M} 50 bzw. das Modul zum Gastransport an einem Gasauslass 49 des Überwachungssystems 100' angeordnet ist. Dies hat - gegenüber der in der Figur 3 gezeigten Variante mit der Pumpe am Gaseinlass des Überwachungssystems - den Vorteil, dass keine Spuren oder Verunreinigungen aus der Pumpe P_{M} 50 in das Überwachungssystem 100', insbesondere in die Sensorik 60 mit einem Kohlenstoffdioxidsensor 64 und einem Sauerstoffsensor 68 gelangen können. Gleiche Elemente in den Figuren 1a, 1b, 1c, 2, 3, 4, 5, 6 sind in den Figuren 1a, 1b, 1c, 2, 3, 4, 5, 6 mit den gleichen Bezugszeichen bezeichnet. Vorzugsweise sind Komponenten, welche für die Kontrolle der Pumpe P_{M} 50 und die Zuführung von Mengen an Gasen notwendig sein können, in unmittelbarer Nähe der Pumpe P_{M} 50 angeordnet. Dazu sind ein Drucksensor 47, ein Durchflusssensor 48 und ein Abschaltventil 57 nahe an der Pumpe P_{M} 50 angeordnet. Der Durchflusssensor 48 dient einer messtechnischen Kontrolle der von der Pumpe P_{M} 50 geförderten Durchflussmenge.

Nach Durchströmung von Pumpe P_{M} 50 und Durchflusssensor 48 gelangt die geförderte Menge an Gas nach außerhalb des Überwachungssystems 100' in die Umgebung 5. Der Drucksensor 47 ist so in Relation zum Abschaltventil 57 stromaufwärts im Gasstrom angeordnet, dass die Druckmessung bei verschlossenem Zustand des Abschaltventils 57 im dann strömungslosen Zustand den Maskendruck in der Atemmaske 20, welcher dann mit dem Druckniveau am Gaseinlass 51 und dem Druckniveau in der Messgasleitung 10 identisch ist, erfassen kann. Alternativ kann der Drucksensor auch am Gasstrom in der Nähe des Gaseinlasses, an der Messgasleitung 10 oder nahe an den Gassensoren 60, 64, 68 angeordnet sein.

Am Gaseinlass 51 ist ein Umschaltventil 55 vorgesehen, welches - in vergleichbarer Weise wie zum Umschaltventil 55 in der Figur 4 beschrieben - eine Umschaltung von Mengen oder Teilmengen an Gasproben zwischen dem Gaseinlass 51 und einem weiteren Gasanschluss 52 ermöglicht. Vorzugsweise ist das Umschaltventil 55 als ein 3/2-Wege-Ventil ausgebildet. Diese Anordnung ermöglicht es, am Gaseinlass 51 mittels der Pumpe P_{M} 50 einerseits Atemgas von der Atemmaske 20 zu der Sensorik 60 zu fördern, es ist aber zudem auch möglich, mittels der Pumpe P_{M} 50 Mengen an Gas oder Gasgemisch durch den weiteren Gaseinlass 52 aus einer Umgebung 5 zu der Sensorik 60 zu fördern und mittels der Sensorik 60 messtechnisch zu erfassen. Das Umschaltventil 55 wird von der Kontrolleinheit 70 kontrolliert. So kann Außenluft von außerhalb des Luftfahrzeuges oder Fluggerätes oder Innenluft aus Kabine oder Cockpit des Fluggerätes über den weiteren Gasanschluss 52 zugeführt werden und abwechselnd - mit Kontrolle der Kontrolleinheit 70 - eine Überwachung von Gaskonzentrationen in der Atemmaske 20, Cockpit, Kabine oder Außenluft ermöglicht sein. Zum Schutz der Sensorik 60 vor Feuchtigkeit und Kondensat, welches aus der Atemmaske 20 durch die Messgasleitung 10 mittels der Pumpe P_{M} 50 der Sensorik 60 zugeführt wird, kann ein Filterelement (HME-Filter) 54 in Serienschaltung in die Messgasleitung 10 bzw. am Auslass des Umschaltventils 55 angeordnet sein.

Anstelle des wie in der Anordnung 100 nach der Figur 3 in Form eines Schaltelementes ausgebildeten Eingabeelementes 80 ist in dieser Ausgestaltung 100' nach der Figur 6 als Eingabeelement ein Beschleunigungssensor 61 vorgesehen, welcher als ein alternatives Betätigungs- oder Eingabeelement zu einer Erfassung von Handbetätigungen des Flugzeugführers ausgebildet und dazu vorgesehen ist. Über dieses alternative Betätigungs- oder Eingabeelement bzw. den Beschleunigungssensor 61 ist es dem Flugzeugführer, Piloten oder Copiloten ermöglicht, bestimmte Ereignisse oder Situationen des Flugbetriebes zu markieren wie auch bestimmte persönliche, beispielsweise gesundheitliche Ereignisse, Situationen oder Symptome, etwa Fieber, Herzrasen oder ein Schwindelgefühl im Zeitverlauf des Einsatzes zu markieren. Diese Markierung kann von der Kontrolleinheit 70 dazu genutzt werden, die Ereignisse oder Situationen mit einer Zeitinformation zu kombinieren und die Kombination von Zeitinformation und Ereignis bzw. Situation dann in einem Datenspeicher 75 abzulegen. Der Datenspeicher 75 kann als flüchtiger oder nichtflüchtiger Speicher (RAM, ROM, EEPROM) ausgebildet sein und sowohl als ein fester Bestandteil oder als entfernbares Speichermodul (USB-Stick, SD-Card) in oder an dem Überwachungssystem 100' angeordnet sein.

Auch kann eine Bereitstellung und/oder ein Austausch der Daten mit einer externen - in den Figuren nicht gezeigten - Auswerteeinheit ermöglicht sein, beispielsweise mittels einer Datenschnittstelle 90 in ähnlicher Ausgestaltung, wie in der Figur 2b gezeigt und beschrieben. Dieses alternative Betätigungs- oder Eingabeelement bzw. der Beschleunigungssensor 61 kann damit dazu dienen, die erfassten Messwerte der Sensorik 60 und die Ereignisse und Situationen des Flugbetriebes um weitere Informationen, die mittels des alternativen Betätigungs- oder Eingabeelements bzw. des Beschleunigungssensors 61 von Flugzeugführer, Piloten oder Copiloten bereitgestellt werden, zu ergänzen und mit einer Zeitinformation, beispielsweise in Form eines Zeitstempels zu versehen. Es ist aber auch eine Ausgestaltung des alternativen Betätigungs- oder Eingabeelements bzw. des Beschleunigungssensors 61 als Paniktaste möglich, welchem dem Flugzeugführer, Piloten oder Copiloten unmittelbar ermöglicht, sich in einer aus seiner Wahrnehmung heraus besonderen Situation, beispielsweise einer Situation mit besonderer objektiver oder subjektiv empfundener Gefahrenlage oder einer Risikosituation, bemerkbar zu machen. Die markierten Messwerte und/oder Ereignisse, Situationen und auch die besonderen Situationen können beispielsweise mittels der Datenschnittstelle 90 nach unmittelbar extern bereitgestellt werden und ggf. - ebenfalls unmittelbar (online) über ein Kommunikationssystem des Luftfahrzeuges oder Fluggerätes - an eine Bodenstation oder an andere Luftfahrzeuge oder Fluggeräte übermittelt werden. Weiterhin ist eine Auswertung der markierten Messwerte und/oder Ereignisse, Situationen und besonderen Situationen nach dem Einsatz im Nachhinein (offline) mittels des Datenspeichers 75 und/oder der Datenschnittstelle 90 ermöglicht.

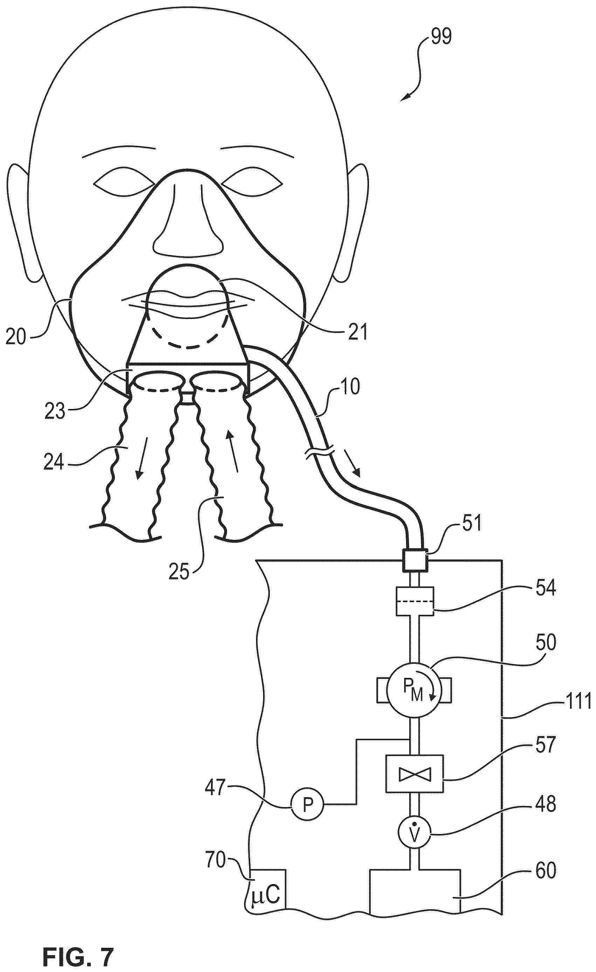

Die Figur 7 zeigt - ausschnittsweise als eine Detailzeichnung des Bereiches um den Gaseinlass 51 und im Unterschied zur Figur 6 - ein Überwachungssystems 111 mit einer Anordnung von Filterelement (HME-Filter) 54, Pumpe P_{M} 50, Sensorik 60, Drucksensor 47, Durchflusssensor 48, Abschaltventil 57 in einer Anordnung am Gaseinlass 51 ohne Umschaltventil zur Umschaltung zwischen einer Überwachung von Atemgasen des Piloten und einer Überwachung der Kabinenluft. Gleiche Elemente in den Figuren 1a, 1b, 1c, 2, 3, 4, 5, 6, 7 sind in den Figuren 1a, 1b, 1c, 2, 3, 4, 5, 6, 7 mit den gleichen Bezugszeichen bezeichnet. Der Drucksensor 47 ist so in Relation zum Abschaltventil 57 stromaufwärts im Gasstrom angeordnet, dass die Druckmessung bei verschlossenem Zustand des Abschaltventils 57 im dann strömungslosen Zustand den Maskendruck in der Atemmaske 20, welcher dann mit dem Druckniveau am Gaseinlass 51 und in der Messgasleitung 10 identisch ist, erfassen kann. Der Drucksensor 47 kann alternativ auch am Gasstrom in der Nähe des Gaseinlasses 51, an der Messgasleitung 10 oder nahe an der Sensorik 60 mit den Gassensoren angeordnet sein.

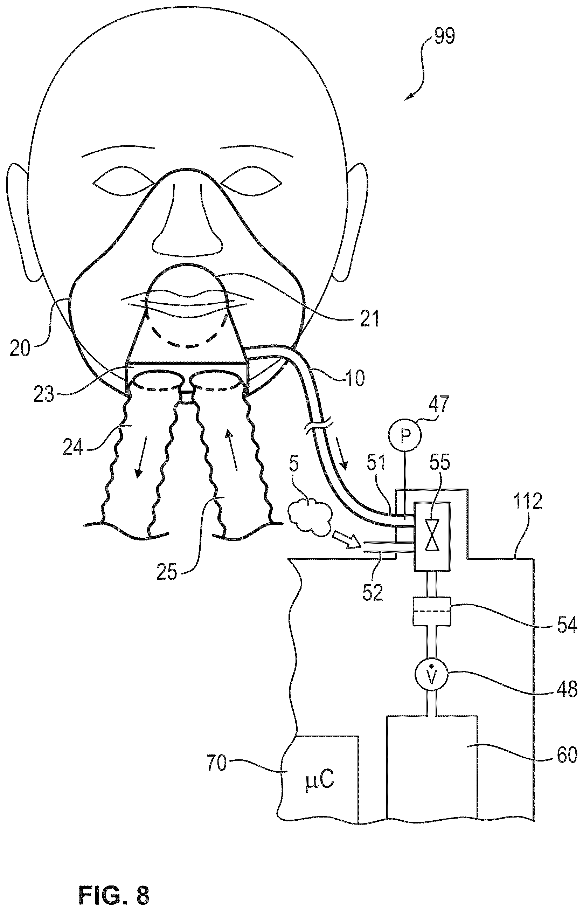

Die Figur 8 zeigt - ausschnittsweise als eine Detailzeichnung des Bereiches um den Gaseinlass 51 und im Unterschied zu den Figuren 6 und 7 - ein Überwachungssystems 111 mit einer Anordnung von Filterelement (HME-Filter) 54, Pumpe P_{M} 50, Sensorik 60, Drucksensor 47 am Gaseinlass 51 und einem Umschaltventil 55 in Ausgestaltung eines 3/2-Wege-Ventils. Gleiche Elemente in den Figuren 1a, 1b, 1c, 2, 3, 4, 5, 6, 7, 8 sind in den Figuren 1a, 1b, 1c, 2, 3, 4, 5, 6, 7, 8 mit den gleichen Bezugszeichen bezeichnet. Das Umschaltventil 55 kann den Weg für Gasmengen aus einer Umgebung 5, etwa der Kabine, zur Sensorik 60 freigeben und damit eine Kabinenluftüberwachung ermöglichen. Zugleich verschließt das Umschaltventil den Weg für Gasmengen aus der Atemmaske 20. In dieser Ausgestaltung nach der Figur 8 weist das Umschaltventil 55 neben der Umschaltung zwischen der Messung von Atemgasen und Kabinenluft auch eine Wirkungsweise als Abschaltventil für die Durchführung eines Messmanövers zur Druckbestimmung in der Atemmaske auf. Der Drucksensor 47 ist so in Relation zum Umschaltventil 55 und der Messgasleitung 10 am Gaseinlass 51 angeordnet, dass die Druckmessung im Zustand des Umschaltventils 55 mit Kabinenluftüberwachung den Maskendruck in der Atemmaske 20, welcher dann mit dem Druckniveau am Gaseinlass 51 und in der Messgasleitung 10 identisch ist, erfassen kann. Das Umschaltventil 55 ermöglicht, zwischen Überwachung von Atemgasen des Piloten und Überwachung der Kabinenluft umzuschalten.

### BEZUGSZEICHENLISTE

- 5: Umgebung, Atmosphäre, Außenluft, Cockpit oder Kabine
- 10: Messgasleitung
- 20: Atemmaske, Messort
- 21: Gasanschluss an Atemmaske
- 24, 25: Schlauchleitungen
- 23, 23': Anschlusselement
- 29: Ausatemventil
- 40: Bedienungselemente
- 44, 45: Anzeigeelemente
- 46: drahtlose Schnittstelle, Funkschnittstelle
- 47: Drucksensor
- 48: Durchflusssensor (Flowsensor, Delta-P-Sensor)
- 49: Gasauslass
- 50: Modul zur Gasförderung, Pumpe P_{M}
- 51: Gaseinlass
- 52, 53: weiterer Gasanschluss
- 54: Filterelement (HME-Filter)
- 55: Umschaltventil, (3/2-Wege-Ventil), Ventilmodul
- 56: weitere Pumpe P_{A}
- 57: Abschalt-Ventil (Flow-Lock-Valve)
- 58: Höhensensor (Altimeter)
- 59, 59': Feuchtesensor
- 60: Sensorik
- 61: Beschleunigungssensor
- 62: Kompass-Sensor
- 63: Gyro-Sensor
- 64, 64': Kohlenstoffdioxidsensor
- 65: weiterer Gassensor
- 66: Sensor
- 67, 67': Drucksensor
- 68, 68': Sauerstoffsensor
- 69, 69': Temperatursensor
- 70: Kontrolleinheit
- 80: Eingabeelement
- 90: Datenschnittstelle
- 99: Person, Pilot, Flugzeugführer
- 100, 100': Überwachungssystem
- 108, 109, 110, 111, 112: Überwachungssystem
- 200: Ablauf des Messmanövers
- 201: Beginn, START
- 202: Pumpe: Deaktivierung
- 203: Abschaltventil: Ventil schließen
- 204: erste Druckmessung: statisches Druckniveau
- 205: Abschaltventil: Ventil öffnen
- 206: Pumpe: Aktivierung
- 207: Flowmessung
- 208: weitere Druckmessung: dynamisches Druckniveau
- 209: Bestimmung des Wertes des aktuellen Druckabfalls
- 210: Ende, STOP

## Patentansprüche

1. Überwachungssystem (100, 100', 108, 109, 110, 111, 112) zur Überwachung einer Gaszusammensetzung von Luft Atemluft oder Atemgasen in Luftfahrzeugen
- mit einer Kontrolleinheit (70) zu einer zu einer Kontrolle des Überwachungssystems,
- mit einem Bedienelement (44) zu einer Bedienung des Überwachungssystems
- mit einer Sensorik (60) mit mindestens einem Gassensor (60),
**dadurch gekennzeichnet, dass**
- die Kontrolleinheit (70) ausgebildet ist, einen Ablauf einer messtechnischen Überwachung der Gaszusammensetzung von Luft, Atemluft oder Atemgasen in Luftfahrzeugen oder Fluggeräten zu organisieren,
zu kontrollieren, zu steuern oder zu regeln,
- an dem Bedienelement (44) ein Beschleunigungssensor (61)
als ein Eingabeelement (80) angeordnet ist,
- die Kontrolleinheit (70) ausgebildet ist,
Überschreitungen mindestens eines Schwellenwertes
für mindestens zwei vorbestimmte Zeitdauern oder für mindestens zwei Krafteinwirkungen oder eine Folgen von mehreren Krafteinwirkungen auf den Beschleunigungssensor (61, 80) zu erfassen
und als Eingabeaktivitäten (44) des Piloten oder Copiloten (99) zu erkennen, und so dem Luftfahrzeugführers (99) mittels des als Beschleunigungssensor (61) ausgebildeten Eingabeelementes (80) zu ermöglichen,
bestimmte Situationen, definierte Aktionen oder Zustände
an dem Überwachungssystem
zu initiieren, zu annotieren, zu triggern, zu starten oder zu beenden.

2. Überwachungssystem (100, 100', 108, 109, 110, 111, 112)
nach Anspruch 1, **dadurch gekennzeichnet, dass**
das Überwachungssystem (100, 100', 108, 109, 110, 111, 112) ein Modul (50) zum Gastransport mit einer Pumpe P_{M} aufweist, welches zu einer Förderung von Mengen oder Teilmengen an Atemgas oder Atemluft von einem Messort (20) zu der Sensorik (60) ausgebildet ist.

3. Überwachungssystem (100, 100', 108, 109, 110, 111, 112)
nach Anspruch 2, **dadurch gekennzeichnet, dass**
das Modul (50) zum Gastransport, vorzugsweise mit Hilfe einer Messgasleitung (10) pneumatisch und/oder fluidisch mit dem Messort (20)
- insbesondere mit einem Gasanschluss (21) an einer Atemmaske (20) -
gasführend verbunden ist.

4. Überwachungssystem (100, 100', 108, 109, 110, 111, 112)
nach Anspruch 2 oder nach Anspruch 3, **dadurch gekennzeichnet, dass**
die Kontrolleinheit (70) zu einer Kontrolle des Moduls (50) zum Gastransport ausgebildet ist.

5. Überwachungssystem (100, 100', 108, 109, 110, 111, 112)
nach einem der vorhergehenden Ansprüche, **dadurch gekennzeichnet, dass**
die Kontrolleinheit (70) ausgebildet ist, bei der Kontrolle des Ablaufs der messtechnischen Überwachung mindestens einen Umgebungsparameter und/oder mindestens einen situativen Parameter, insbesondere Initiierungen (44) des Piloten oder Copiloten (99) mittels des Eingabeelementes (80, 61) mit zu berücksichtigen und/oder in den Ablauf mit einzubeziehen.

6. Überwachungssystem (100, 100', 108, 109, 110, 111, 112)
nach einem der vorhergehenden Ansprüche, **dadurch gekennzeichnet, dass**
das Überwachungssystem eine Datenschnittstelle (90) zu einem Empfang und/oder einer Bereitstellung von Umgebungsparametern und/oder situativen Parametern aufweist.

7. Überwachungssystem (100, 100', 108, 109, 110, 111, 112)
nach einem der vorhergehenden Ansprüche, **dadurch gekennzeichnet, dass**
die Sensorik (60) weitere Sensoren (61, 62, 63, 64, 64', 65, 67, 67' 58, 58'),
- insbesondere den Beschleunigungssensor (61) -
zu einer Bestimmung und/oder messtechnischen Erfassung von Umgebungsparametern und/oder zu einer Bestimmung und/oder messtechnischen Erfassung von situativen Parametern aufweist und
zu einer Bereitstellung der Umgebungsparameter
und/oder situativen Parameter ausgebildet ist.

8. Überwachungssystem (100, 100', 108, 109, 110, 111, 112)
nach einem der vorhergehenden Ansprüche, **dadurch gekennzeichnet, dass**
- das Überwachungssystem und/oder die Kontrolleinheit (70) zu einer Bestimmung und/oder Erkennung einer Alarmsituation und zu einer Organisation einer Alarmierung oder Alarmgabe und/oder zu einer Bereitstellung eines Alarmsignals ausgebildet ist,
- die Kontrolleinheit (70) ausgebildet ist, bei der Organisation der Alarmierung oder Alarmgabe und/oder bei der Bereitstellung des Alarmsignals einen Umgebungsparameter und/oder einen situativen Parameter mit zu berücksichtigen und/oder in die Organisation der Alarmierung mit einzubeziehen.

9. Überwachungssystem (100, 100', 108, 109, 110, 111, 112)
nach einem der vorhergehenden Ansprüche, **dadurch gekennzeichnet, dass**
in oder an dem Überwachungssystem mindestens ein Anzeigeelement
zu einer Anzeige von
- Ereignissen, Situationen, Statusdaten, aktuellen Messwerten,
- zeitlich zurück liegenden Messwerten,
- von Messwerten abgeleitete Messgrößen, wie beispielsweise Maximal- oder Minimalwerte, Mittelwerte, Trends, Statistiken, Ereignissen, Alarmsituationen
angeordnet ist oder dem Überwachungssystem zugeordnet ist.

10. Überwachungssystem (100, 100', 108, 109, 110, 111, 112)
nach einem der vorhergehenden Ansprüche, **dadurch gekennzeichnet, dass**
in oder an dem Überwachungssystem ein Datenspeicher zur Speicherung von Messwerten von, von den Messwerten abgeleiteten Messgrößen wie beispielsweise Maximal- oder Minimalwerte, Mittelwerte, Trends, Statistiken, Ereignisse, Alarmsituationen angeordnet oder dem Überwachungssystem zugeordnet ist.

11. Überwachungssystem (100, 100', 108, 109, 110, 111, 112)
nach einem der vorhergehenden Ansprüche, **dadurch gekennzeichnet, dass**
die Kontrolleinheit (70) ausgebildet ist,
bei einer Durchführung der Signalverarbeitung und/oder Signalfilterung der Messwerte der Sensorik einen Umgebungsparameter und/oder einen situativen Parameter mit zu berücksichtigen und/oder mit in eine Anpassung der Signalverarbeitung einzubeziehen.

12. Überwachungssystem (100, 100', 108, 109, 110, 111, 112)
nach einem der vorhergehenden Ansprüche, **dadurch gekennzeichnet, dass**
die Kontrolleinheit (70) ausgebildet ist,
bei der Organisation der Alarmierung vorbestimmte Schwellenwerte zu verwenden, welche für bestimmte Werte von Gaskonzentrationen, insbesondere Konzentrationen an Sauerstoff, Kohlenstoffdioxid oder Kohlenstoffmonoxid im Datenspeicher des Überwachungssystems hinterlegt sind.
